# EUROPÄISCHE PATENTSCHRIFT

(11) **EP 1 797 093 B1**
(45) Veröffentlichungstag und Bekanntmachung des Hinweises auf die Patenterteilung: **30.05.2012**
(21) Anmeldenummer: 05781895.7
(22) Anmeldetag: 09.09.2005
(51) Int. Cl.: C07D 473/04, A61P 3/10, A61K 31/4985

(54) **NEUE 3-METHYL-7-BUTINYL-XANTHINE, DEREN HERSTELLUNG UND DEREN VERWENDUNG ALS ARZNEIMITTEL**
NOVEL 3-METHYL-7-BUTINYL-XANTHINES, PRODUCTION THEREOF, AND USE THEREOF AS MEDICAMENTS
NOUVELLES 3-METHYL-7-BUTINYL-XANTHINES, LEUR PRODUCTION ET LEUR UTILISATION COMME MEDICAMENTS

(30) Priorität: 14.09.2004 DE 102004044221
(43) Veröffentlichungstag der Anmeldung: 20.06.2007
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: LANGKOPF, Elke, 88447 WARTHAUSEN (DE); ECKHARDT, Matthias, 88400 BIBERACH (DE); HIMMELSBACH, Frank, 88441 MITTELBIBERACH (DE); TADAYYON, Mohammad, Hertford SG14 1HQ (GB); THOMAS, Leo, 88400 BIBERACH (DE); LOTZ, Ralf, R., H., 88433 SCHEMMERHOFEN (DE)
(74) Vertreter: Hammann, Heinz
(86) Internationale Anmeldenummer: PCT/EP2005/009712
(87) Internationale Veröffentlichungsnummer: WO 2006/029769

(56) Entgegenhaltungen:
- WO-A-02/068420
- WO-A-2004/018467
- WO-A-2004/041820
- WO-A-2004/046148
- WO-A-2005/082906

## Beschreibung

Gegenstand der vorliegenden Erfindung sind neue substituierte Xanthine der allgemeinen Formel 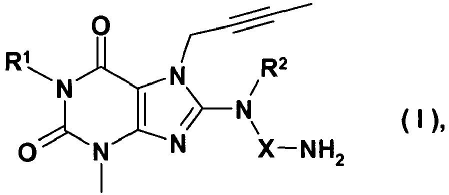 deren Tautomere, deren Enantiomere, deren Diastereomere, deren Gemische und deren Salze, insbesonders deren physiologisch verträglichen Salze mit anorganischen oder organischen Säuren, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

Verwandte Xanthine werden in der internationalen Anmeldung WO 02/068420 beschrieben.

Die internationalen Anmeldungen WO 2004/018467, WO 2004/041820, WO 2004/046148 und WO 2005/082906 beschreiben weitere Verbindungen mit einer Hemmwirkung auf die Aktivität von DPP-IV.

In der obigen Formel I bedeuten
R¹ eine Heteroarylmethyl- oder Heteroarylethylgruppe,
eine Heteroarylcarbonylmethylgruppe oder
eine Arylprop-2-enyl- oder Heteroarylprop-2-enylgruppe, in denen die Propenylkette durch 1 bis 4 Fluoratome oder eine Cyan-, C₁₋₃-Alkyloxy-carbonyl- oder Nitrogruppe substituiert sein kann,
R² eine C₁₋₄-Alkylgruppe, welche geradkettig oder verzweigt sein kann, und
X eine -CH₂CH₂-Gruppe, welche gegebenenfalls durch ein oder zwei C₁₋₃-Alkylgruppen, die gleich oder verschieden sein können, substituiert sein kann,
wobei unter den bei der Definition der vorstehend genannten Reste erwähnten Arylgruppen Phenyl- oder Naphthylgruppen zu verstehen sind, welche unabhängig voneinander durch Rₕ mono-, di- oder trisubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, C₁₋₃-Alkoxycarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Phenyl-, Morpholinyl-, Hydroxy-, C₁₋₃-Alkyloxy-, Difluormethoxy- oder Trifluormethoxygruppe darstellt, oder zwei Rₕ an zwei benachbarten Kohlenstoffatomen des Aromaten zusammen eine C₃₋₅-Alkylenkette bilden, wobei in der Alkylenkette ein oder zwei Methylengruppen unabhängig voneinander gegen Sauerstoffatome oder Carbonylgruppen substituiert sein können, und in denen zusätzlich jedes Wasserstoffatom durch ein Fluoratom ersetzt sein kann,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Phenanthridinyl-, Chinolinyl- oder Isochinolinylgruppe zu verstehen ist,
oder eine Pyrrolyl-, Furanyl-, Thienyl-, Imidazolyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Phenanthridinyl-, Chinolinyl-oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1H-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl-, Chinolinonyl-, Imidazo-chinolinyl-, Chinazolinonyl-, Benzonaphthiridinyl- oder 3,4-Dihydro-3-oxo-2H-benzo-[1,4]oxazinyl-Gruppe zu verstehen ist,
und die vorstehend erwähnten Heteroarylgruppen durch Rₕ mono- oder disubstituiert sein können, wobei die Substituenten gleich oder verschieden sein können und Rₕ wie vorstehend erwähnt definiert ist,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische, und deren Salze.

Die bei der Definition der vorstehend erwähnten Reste erwähnten Amino- und Iminogruppen können durch einen in-vivo abspaltbaren Rest substituiert sein. Derartige Gruppen werden beispielsweise in der WO 98/46576 beschrieben.

Unter einem von einer Imino- oder Aminogruppe in-vivo abspaltbaren Rest ist beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkoxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁₋₁₆-Alkoxycarbonyl-oder C₁₋₁₆-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.-Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy-oder Hexadecylcarbonyloxygruppe, eine Phenyl-C₁₋₆-alkoxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkoxycarbonyl-, C₁₋₃-Alkoxy-C₂₋₄-alkoxy-C₂₋₄-alkoxycarbonyl-, Rₚ-CO-O-(R_{q}CRᵣ)-O-CO-, C₁₋₆-Alkyl-CO-NH-(RₛCRₜ)-O-CO- oder C₁₋₆-Alkyl-CO-O-(RₛCRₜ)-(RₛCRₜ)-O-CO-Gruppe, in denen Rₚ bis Rᵣ wie vorstehend erwähnt definiert sind,
Rₛ und Rₜ, die gleich oder verschieden sein können, Wasserstoffatome oder C₁₋₃-Alkylgruppen darstellen,
zu verstehen.

Des Weiteren schließen die in den vor- und nachstehenden Definitionen erwähnten gesättigten Alkyl- und Alkoxyteile, die mehr als 2 Kohlenstoffatome enthalten, soweit nichts Anderes erwähnt wurde, auch deren verzweigte Isomere wie beispielsweise die Isopropyl-, tert.-Butyl-, Isobutylgruppe etc. ein.

Für R¹ kommt beispielsweise die Bedeutung einer 5-Cyanfuranylmethyl-, Oxazolylmethyl-, Isoxazolylmethyl-, 5-Methoxycarbonylthienylmethyl-, Pyridinylmethyl-, 3-Cyanpyridin-2-ylmethyl-, 5-Cyanpyridin-2-ylmethyl-, 6-Cyanpyridin-2-ylmethyl-, 4-Cyanpyridin-3-ylmethyl, 6-Fluorpyridin-2-ylmethyl-, Pyrimidin-2-yl-, 4-Methylpyrimidin-2-yl-, 4,6-Dimethylpyrimidin-2-yl-, 3-(2-Cyanphenyl)-prop-2-enyl-, 3-(2-Nitrophenyl)-prop-2-enyl-, 3-(Pyridin-2-yl)-prop-2-enyl-, 3-(Pentafluorphenyl)-prop-2-enyl-, 1-Methyl-benzotriazol-5-ylmethyl-, Chinolin-1-ylmethyl-, Chinolin-2-ylmethyl-, Chinolin-6-ylmethyl-, Chinolin-7-ylmethyl-, 3-Cyanchinolin-2-ylmethyl-, 4-Cyanchinolin-2-ylmethyl-, 8-Cyanchinolin-2-ylmethyl-, 8-Cyanchinolin-7-ylmethyl-, Isochinolin-1-ylmethyl-, 4-Cyanisochinolin-1-ylmethyl-, 1-Cyanisochinolin-3-ylmethyl-, 4-Cyanisochinolin-3-ylmethyl-, 3-Methylisochinolin-1-ylmethyl-, Chinazolin-2-ylmethyl-, 4-Methylchinazolin-2-ylmethyl-, 4-Cyanchinazolin-2-ylmethyl-, 4-Aminochinazolin-2-ylmethyl-, 4-Morpholin-4-ylchinazolin-2-ylmethyl-, [1,5]Naphthyridin-2-ylmethyl-, [1,5]Naphthyridin-3-ylmethyl-, [1,8]Naphthyridin-2-ylmethyl-, Phenanthridin-6-ylmethyl-, Chinoxalin-2-ylmethyl-, Chinoxalin-6-ylmethyl- oder 2,3-Dimethyl-chinoxa-lin-6-ylmethylgruppe in Betracht.

Bevorzugt sind diejenigen Verbindungen der allgemeinen Formel I, in denen
R¹ wie oben erwähnt definiert ist,
R² eine Methyl- oder Ethylgruppe und
X eine -CH₂CH₂-Gruppe, welche gegebenenfalls durch ein oder zwei Methyl- oder Ethylgruppen substituiert sein kann, wobei die Substituenten gleich oder verschieden sein können, bedeuten
deren Tautomere, Enantiomere, Diastereomere, deren Gemische und deren Salze.

Besonders bevorzugt sind diejenigen Verbindungen der allgemeinen Formel I, in denen
R¹ eine Phenylprop-2-enyl-, Pyridinylmethyl-, Pyrimidinylmethyl-, Chinolinylmethyl-, Chinolinonylmethyl-, Imidazochinolinylmethyl-, Isochinolinylmethyl-, Chinazolinylmethyl-, Chinazolinonylmethyl-, Chinoxalinylmethyl-, Phenanthridinylmethyl-, Naphthyridinylmethyl-, Benzonaphthiridinylmethyl-, Imidazopyridinylmethyl- oder Benzotriazolylmethylgruppe, die jeweils durch ein oder zwei Fluor-, Chlor-, Bromatome oder ein oder zwei Cyan-, Nitro-, Amino-, C₁₋₃-Alkyl-, C₁₋₃-Alkyloxy-, Phenyl-und Morpholinylgruppen substituiert sein können, wobei die Substituenten gleich oder verschieden sind,
R² eine Methylgruppe und
X eine -CH₂CH₂-Gruppe, welche gegebenenfalls durch ein oder zwei Methylgruppen substituiert sein kann, bedeuten,
deren Tautomere, Enantiomere, Diastereomere, deren Gemische und deren Salze.

Ganz besonders bevorzugt sind diejenigen Verbindungen der allgemeinen Formel I, in denen
R¹ eine Pyridinylmethyl-, Pyrimidinylmethyl-, Chinolinylmethyl-, Isochinolinylmethyl-, Chinazolinylmethyl-, Chinoxalinylmethyl-, oder Naphthyridinylmethyl-, welche jeweils mit ein oder zwei Cyan- oder Methylgruppen substituiert sein können,
R² eine Methylgruppe und
X eine -CH(CH₃)-CH₂-Gruppe, eine -CH₂-CH(CH₃)-Gruppe oder eine -CH₂-C(CH₃)₂-Gruppe bedeuten,
deren Tautomere, Enantiomere, Diastereomere, deren Gemische und deren Salze';
insbesondere jedoch diejenigen Verbindungen der allgemeinen Formel I, in denen
R¹ eine Pyridinylmethyl-, Isochinolinylmethyl-, Chinazolinylmethyl-, Chinoxalinylmethyl- oder Naphthyridinylmethylgruppe, welche jeweils mit einer Cyan-oder Methylgruppe substituiert sein können,
R² eine Methylgruppe und
X eine -CH(CH₃)-CH₂-Gruppe, eine -CH₂-CH(CH₃)-Gruppe oder eine -CH₂-C(CH₃)₂-Gruppe, wobei jeweils das rechts stehende Kohlenstoffatom mit der endständigen Aminogruppe verknüpft ist, bedeuten,
deren Tautomere, Enantiomere, Diastereomere, deren Gemische und deren Salze.

Beispielsweise seien folgende bevorzugte Verbindungen erwähnt:
(a) 1-[(3-Methyl-isochinolin-1-yl)methyl]-3-methyl-7-(2-butin-1-yl)-8-[(2-aminoethyl)-methylamino]-xanthin
(b) 1-[(4-Methyl-chinazolin-2-yl)methyl]-3-methyl-7-(2-butin-1-yl)-8-[(2-amino-ethyl)-methylamino]-xanthin
(c) 1-[(3-Methyl-isochinolin-1-yl)methyl]-3-methyl-7-(2-butin-1-yl)-8-[(2-amino-2-methyl-propyl)-methylamino]-xanthin
(d) 1-[(4-Methyl-chinazolin-2-yl)methyl]-3-methyl-7-(2-butin-1-yl)-8-[(2-amino-2-methyl-propyl)-methylamino]-xanthin
(e) 1-[(3-Methyl-isochinolin-1-yl)methyl]-3-methyl-7-(2-butin-1-yl)-8-[(S)-(2-amino-propyl)-methylamino]-xanthin
(f) 1-[(3-Methyl-isochinolin-1-yl)methyl]-3-methyl-7-(2-butin-1-yl)-8-[(R)-(2-amino-propyl)-methylamino]-xanthin
(g) 1-[(4-Methyl-chinazolin-2-yl)methyl]-3-methyl-7-(2-butin-1-yl)-8-[(S)-(2-amino-propyl)-methylamino]-xanthin
(h) 1-[(4-Methyl-chinazolin-2-yl)methyl]-3-methyl-7-(2-butin-1-yl)-8-[(R)-(2-amino-propyl)-methylamino]-xanthin
(i) 1-[(3-Methyl-isochinolin-1-yl)methyl]-3-methyl-7-(2-butin-1-yl)-8-[(R)-(2-amino-1-methyl-ethyl)-methylamino]-]-xanthin
(j) 1-[(3-Methyl-isochinolin-1-yl)methyl]-3-methyl-7-(2-butin-1-yl)-8-[(S)-(2-amino-1-methyl-ethyl)-methylamino]-]-xanthin
(l) 1-[([1,5]Naphthyridin-2-yl)methyl]-3-methyl-7-(2-butin-1-yl)-8-[(S)-(2-amino-propyl)-methylamino]-xanthin
(m) 1-[(Chinoxalin-6-yl)methyl]-3-methyl-7-(2-butin-1-yl)-8-[(S)-(2-amino-propyl)-methylamino]-xanthin
(n) 1-[(3-Cyano-pyridin-2-yl)methyl]-3-methyl-7-(2-butin-1-yl)-8-[(S)-(2-amino-propyl)-methylamino]-xanthin
(o) 1-[(4-Cyano-isochinolin-3-yl)methyl]-3-methyl-7-(2-butin-1-yl)-8-[(*S*)-(2-amino-propyl)-methylamino]-xanthin
(q) 1-[(3-Cyano-pyridin-2-yl)methyl]-3-methyl-7-(2-butin-1 -yl)-8-[(2-amino-2-methylpropyl)-methylamino]-xanthin
sowie deren Salze.

Erfindungsgemäß erhält man die Verbindungen der allgemeinen Formel I nach an sich bekannten Verfahren, beispielsweise nach folgenden Verfahren:
a) Umsetzung einer Verbindung der allgemeinen Formel 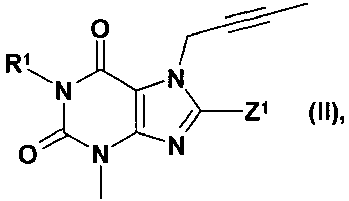 in der
   R¹ wie eingangs erwähnt definiert ist und
   Z¹ eine Austrittsgruppe wie ein Halogenatom, eine substituierte Hydroxy-, Mercapto-, Sulfinyl-, Sulfonyl- oder Sulfonyloxygruppe wie z. B. ein Chlor-, Brom- oder lodatom, eine Methansulfonyl-, Trifluormethansulfonyloxy- oder Methansulfonyloxygruppe darstellt,
   mit einer Verbindung der allgemeinen Formel 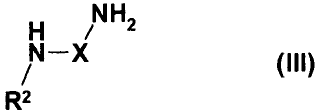 oder 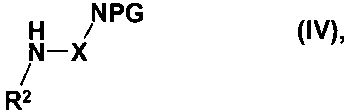
worin R² und X wie eingangs erwähnt definiert sind und NPG eine geschützte oder maskierte Aminofunktionalität darstellt, Derivaten oder Salzen davon.

Als Schutzreste für die Aminogruppe kommen z. B. die Formyl-, Acetyl-, Trifluoracetyl-, Ethoxycarbonyl-, tert.-Butoxycarbonyl-, Allyloxycarbonyl-, Benzyloxycarbonyl-, p-Methoxybenzylcarbonyl-, Benzyl-, Methoxybenzyl-, 2,4-Dimethoxybenzyl, Phthalyl- oder Tetrachlorphthalylgruppe in Betracht. Die Aminogruppe kann aber auch z. B. Teil eines Heteroaromaten wie z. B. 2,5-Dimethylpyrrol sein und aus diesem später wieder frei gesetzt werden.

Die Aminofunktion kann auch in Form einer Carboxygruppe oder eines Derivats davon maskiert sein, die nach einem so genannten Curtius-, Schmidt- oder Hofmann-Abbau in die Aminofunktion überführt werden kann (siehe u.a. J. March, Advanced Organic Reactions, Reactions, Mechanisms, and Structure, 4. Edition, John Wiley & Sons, Chichester/New York/Brisbane/Toronto/Singapore, 1992 und darin zitierte Literatur).

Die Umsetzung wird zweckmäßigerweise in einem Lösungsmittel wie z. B. Isopropanol, Butanol, Tetrahydrofuran, Dioxan, Dimethylformamid, Dimethylsulfoxid, Ethylenglycolmonomethylether, Ethylenglycoldiethylether oder Sulfolan gegebenenfalls in Gegenwart einer anorganischen oder tertiären organischen Base, z. B. Natriumcarbonat, Kaliumcarbonat oder Kaliumhydroxid, einer tertiären organischen Base, z. B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladium- oder Kupferbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel in einem Überschuß von Piperidinderivat unter konventionellem Erwärmen oder im Mikrowellenofen durchgeführt werden.

### b) Entschützung einer Verbindung der allgemeinen Formel

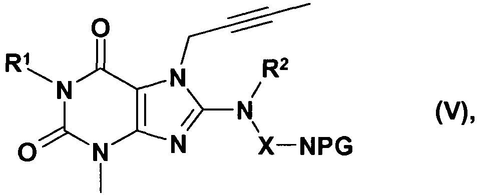

in der R¹, R² und X wie eingangs erwähnt definiert sind und NPG eine geschützte oder maskierte Aminofunktionalität darstellt. Mögliche Schutzgruppen bzw. Maskierungen der Aminofunktion sind unter a) bereits erwähnt worden. Vorzugsweise ist die Aminogruppe mit einem tert.-Butoxycarbonyl- oder Phthalylrest geschützt.

Die Abspaltung des tert.-Butoxycarbonylrestes erfolgt vorzugsweise durch Behandlung mit einer Säure wie Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C. Die Abspaltung des Phthalylrestes erfolgt vorzugsweise in Gegenwart von Hydrazin oder eines primären Amins wie Methylamin, Ethylamin, Ethanolamin oder n-Butylamin in einem Lösungsmittel wie Methanol, Ethanol, Isopropanol, Toluol, Toluol/Wasser oder Dioxan bei Temperaturen zwischen 20 und 120°C.

Bei den vorstehend beschriebenen Umsetzungen können gegebenenfalls vorhandene reaktive Gruppen wie Amino-, Alkylamino- oder Iminogruppen während der Umsetzung durch übliche Schutzgruppen geschützt werden, welche nach der Umsetzung wieder abgespalten werden.

Beispielsweise kommen als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z. B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z. B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z. B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Ethanolamin, Isopropanol, Toluol, Toluol/Wasser oder Dioxan bei Temperaturen zwischen 20 und 120°C.

Die Freisetzung einer Aminofunktion aus 2,5-Dimethylpyrrol erfolgt beispielsweise mit Hydroxylaminhydrochlorid in Gegenwart einer Base wie z. B. Triethylamin in einem geeigneten Lösungsmittel wie einem Alkohol wie z. B. Methanol, Ethanol, Propanol oder Isopropanol oder Wasser oder Gemischen daraus bei Temperaturen zwischen 0 und 150°C, vorzugsweise jedoch bei Raumtemperaturen zwischen 50 und 110°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens zwei asymmetrischen Kohlenstoffatomen auf Grund ihrer physikalisch-chemischen Unterschiede nach an sich bekannten Methoden, z. B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z. B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z. B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z. B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Benzoesäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsstoffe verwendeten Verbindungen der allgemeinen Formeln II-V sind entweder literaturbekannt oder man erhält diese nach an sich literaturbekannten Verfahren (siehe Beispiele I-XI).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP-IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2", erschienen in Proc. Natl. Acad. Sci. Vol. 90, Seiten 5757-5761 (1993), durchgeführt. Der Zellextrakt wurde von in einem Puffer (10 mM Tris HCl, 0.15 M NaCl, 0.04 t.i.u. Aprotinin, 0.5% Nonidet-P40, pH 8.0) solubilisierten Zellen durch Zentrifugation bei 35000 g für 30 Minuten bei 4°C (zur Entfernung von Zelltrümmern) gewonnen.

Der DPP-IV Assay wurde wie folgt durchgeführt:

50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung | DPP IV-Hemmung |
|---|---|
| (Beispiel Nr.) | IC₅₀ [nM] |
| 1 | 4 |
| 1(1) | 2 |
| 2(1) | 3 |
| 2(4) | 2 |
| 2(5) | 4 |
| 2(9) | 4 |
| 2(10) | 3 |
| 2(11) | 3 |
| 2(12) | 2 |

Die erfindungsgemäß hergestellten Verbindungen sind gut verträglich, da beispielsweise nach oraler Gabe von 10 mg/kg der Verbindung des Beispiels 2(4) an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, dass die erfindungsgemäßen Verbindungen zur Prävention oder Behandlung von Krankheiten oder Zuständen wie Diabetes mellitus Typ 1 und Typ 2, Prädiabetes, Verminderung der Glukosetoleranz oder Veränderungen im Nüchternblutzucker, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, dass die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die-erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, dass sie bei jeglicher Art von Verletzung oder Beeinträchtigung im Gastrointestinaltrakt eingesetzt werden können wie auch z.B. bei Kolitiden und Enteriden. Darüberhinaus wird erwartet, dass DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neurdegenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570) und -Antagonisten, PPAR-gamma/alpha Modulatoren (z.B. KRP 297), PPAR-gamma/alpha/delta Modulatoren, AMPK-Aktivatoren, ACC1 und ACC2 Inhibitoren, DGAT-Inhibitoren, SMT3-Rezeptor-Agonisten, 11ß-HSD-Inhibitoren, FGF19-Agonisten oder -Mimetika, alpha-Glucosidasehemmer (z.B. Acarbose, Voglibose), andere DPPIV Inhibitoren, alpha2-Antagonisten, Insulin und Insulinanaloga, GLP-1 und GLP-1 Analoga (z.B. Exendin-4) oder Amylin. Daneben sind Kombinationen mit SGLT2-Inhibitoren wie T-1 095 oder KGT-1251 (869682), Inhibitoren der Proteintyrosinphosphatase 1, Substanzen, die eine deregulierte Glucoseproduktion in der Leber beeinflussen, wie z.B. Inhibitoren der Glucose-6-phosphatase oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder LXRalpha Antagonisten, LXRbeta Agonisten oder LXRalpha/beta Regulatoren oder Wirkstoffen zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder ß₃-Agonisten wie SB-418790 oder AD-9677 ebenso wie Agonisten des 5HT2c Rezeptors möglich.

Daneben ist eine Kombination mit Medikamenten zur Beeinflussung des Bluthochdrucks wie z.B. All Antagonisten oder ACE Inhibitoren, Diuretika, ß-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 1-[(3-Methyl-isochinolin-1-yl)methyl]-3-methyl-7-(2-butin-1-yl)-8-brom-xanthin

Ein Gemisch aus 6.97 g 3-Methyl-7-(2-butin-1-yl)-8-brom-xanthin, 4.94 g 1-Chlormethyl-3-methyl-isochinolin und 6.64 g Kaliumcarbonat in 56 ml N-Methylpyrrolidon wird 3.5 Stunden bei 75°C gerührt. Zur Aufarbeitung wird es mit 80 ml Wasser versetzt. Der entstandene Niederschlag wird abgesaugt, mit Wasser gewaschen und getrocknet.
Ausbeute: 9.11 g (86 % der Theorie)
R_{f}-Wert: 0.45 (Kieselgel, Essigester/Petrolether = 1:1)

Analog Beispiel I werden folgende Verbindungen erhalten:
(1) 1-[(4-Methyl-chinazolin-2-yl)methyl]-3-methyl-7-(2-butin-1-yl)-8-brom-xanthin Massenspektrum (ESI⁺): m/z = 453, 455 [M+H]⁺
(2) 1-(2-Cyano-benzyl)-3-methyl-7-(2-butin-1-yl)-8-brom-xanthin (Durchführung in Gegenwart von Kaliumcarbonat in Dimethylsulfoxid bei 80-90°C) R_{f}-Wert: 0.80 (Kieselgel, Essigester)
   Massenspektrum (ESI⁺): m/z = 412, 414 [M+H]⁺
(3) 1-[([1,5]Naphthyridin-2-yl)methyl]-3-methyl-7-(2-butin-1-yl)-8-brom-xanthin (Durchführung in Gegenwart von Kaliumcarbonat in N,N-Dimethylformamid bei 80°C) R_{f}-Wert: 0.39 (Kieselgel, Essigester)
   Massenspektrum (ESI⁺): m/z = 439, 441 [M+H]⁺
(4) 1-[(Chinoxalin-6-yl)methyl]-3-methyl-7-(2-butin-1-yl)-8-brom-xanthin (Durchführung in Gegenwart von Kaliumcarbonat in Dimethylsulfoxid bei 80-85°C)
   R_{f}-Wert: 0.55 (Kieselgel, Essigester)
   Massenspektrum (ESI⁺): m/z = 439, 441 [M+H]⁺
(5) 1-[(3-Cyano-pyridin-2-yl)methyl]-3-methyl-7-(2-butin-1-yl)-8-brom-xanthin R_{f}-Wert: 0.65 (Kieselgel, Essigester)
(6) 1-[(4-Cyano-isochinolin-3-yl)methyl]-3-methyl-7-(2-butin-1-yl)-8-{(*S*)-[2-(tert.-butoxycarbonylamino)-propyl]-methylamino}-xanthin
   R_{f}-Wert: 0.60 (Kieselgel, Essigester)
   Massenspektrum (ESI⁺): m/z = 571 [M+H]⁺
(7) 1-(4-Cyano-benzyl)-3-methyl-7-(2-butin-1-yl)-8-brom-xanthin (Durchführung in Gegenwart von Kaliumcarbonat in N-Methylpyrrolidon bei 85°C) Massenspektrum (ESI⁺): m/z = 412, 414 [M+H]⁺
(8) 1-(3-Cyano-benzyl)-3-methyl-7-(2-butin-1-yl)-8-brom-xanthin (Durchführung in Gegenwart von Kaliumcarbonat in N-Methylpyrrolidon bei 85°C) Massenspektrum (ESI⁺): m/z = 412, 414 [M+H]⁺

### Beispiel II

### 1-[(3-Methyl-isochinolin-1-yl)methyl]-3-methyl-7-(2-butin-1-yl)-8-{[2-(tert.-butoxy-carbonylamino)-2-methyl-propyl-methylamino}-xanthin

Zu 770 mg 1-[(3-Methyl-isochinolin-1-yl)methyl]-3-methyl-7-(2-butin-1-yl)-8-[2-(tert.-butoxycarbonylamino)-2-methyl-propylamino]-xanthin in 4 ml N,N-Dimethylformamid werden unter Eiskühlung 64 mg Natriumhydrid (60 % in Mineralöl) gegeben. Nach fünf Minuten wird das Eisbad entfernt und das Reaktionsgemisch wird 15 Minuten bei Raumtemperatur gerührt. Anschließend werden unter Eisbadkühlung 97 µl Methyliodid zugegeben. Nach einer halben Stunde ist die Umsetzung vollständig. Das Reaktionsgemisch wird mit etwas verdünnter Natriumcarbonat-Lösung versetzt und mit Essigester extrahiert. Die vereinigten organischen Phasen werden mit gesättigter Natriumchlorid-Lösung gewaschen, über Magnesiumsulfat getrocknet und eingeengt. Das Rohprodukt wird mit tert.-Butylmethylether verrieben, abgesaugt, mit wenig tert.-Butylmethylether gewaschen und getrocknet.
Ausbeute: 655 mg (83 % der Theorie)
R_{f}-Wert: 0.60 (Kieselgel, Essigester/Petrolether = 3:2)
Massenspektrum (ESI⁺): m/z = 574 [M+H]⁺

Analog Beispiel II werden folgende Verbindungen erhalten:
(1) 1-[(4-Methyl-chinazolin-2-yl)methyl]-3-methyl-7-(2-butin-1-yl)-8-{[2-(tert.-butoxycarbonylamino)-2-methyl-propyl]-methylamino}-xanthin
   R_{f}-Wert: 0.66 (Kieselgel, Essigester)
   Massenspektrum (ESI⁺): m/z = 575 [M+H]⁺
(2) 1-[(3-Methyl-isochinolin-1-yl)methyl]-3-methyl-7-(2-butin-1-yl)-8-{(*S*)-[2-(tert.-butoxycarbonylamino)-propyl]-methylamino}-xanthin
   (Durchführung mit Kalium-tert.-butylat in Dimethylsulfoxid)
   R_{f}-Wert: 0.45 (Kieselgel, Methylenchlorid/Methanol = 95:5)
   Massenspektrum (ESI⁺): m/z = 560 [M+H]⁺
(3) 1-[(3-Methyl-isochinolin-1-yl)methyl]-3-methyl-7-(2-butin-1-yl)-8-{(*R*)-[2-(tert.-butoxycarbonylamino)-propyl]-methylamino}-xanthin
   (Durchführung mit Kalium-tert.-butylat in Dimethylsulfoxid)
   R_{f}-Wert: 0.70 (Kieselgel, Essigester)
   Massenspektrum (ESI⁺): m/z = 560 [M+H]⁺
(4) 1-[(4-Methyl-chinazolin-2-yl)methyl]-3-methyl-7-(2-butin-1-yl)-8-{(*S*)-[2-(tert.-butoxycarbonylamino)-propyl]-methylamino}-xanthin
   (Durchführung mit Kalium-tert.-butylat in Dimethylsulfoxid)
   R_{f}-Wert: 0.40 (Kieselgel, Essigester)
   Massenspektrum (ESI⁺): m/z = 561 [M+H]⁺
(5) 1-[(4-Methyl-chinazolin-2-yl)methyl]-3-methyl-7-(2-butin-1-yl)-8-{(*R*)-[2-(tert.-butoxycarbonylamino)-propyl]-methylamino}-xanthin
   (Durchführung mit Kalium-tert.-butylat in Dimethylsulfoxid)
   R_{f}-Wert: 0.30 (Kieselgel, Methylenchlorid/Methanol = 95:5)
   Massenspektrum (ESI⁺): m/z = 561 [M+H]⁺
(6) 1-[(3-Methyl-isochinolin-1-yl)methyl]-3-methyl-7-(2-butin-1-yl)-8-{(*R*)-[2-(tert.-butoxycarbonylamino)-1-methyl-ethyl]-methylamino}-xanthin
   (Durchführung mit Kalium-tert.-butylat in Dimethylsulfoxid)
   R_{f}-Wert: 0.55 (Kieselgel, Methylenchlorid/Methanol = 95:5)
   Massenspektrum (ESI⁺): m/z = 560 [M+H]⁺
(7) 1-[(3-Methyl-isochinolin-1-yl)methyl]-3-methyl-7-(2-butin-1-yl)-8-{(*S*)-[2-(tert.-butoxycarbonylamino)-1-methyl-ethyl]-methylamino}-xanthin
   (Durchführung mit Kalium-tert.-butylat in Dimethylsulfoxid)
   Massenspektrum (ESI⁺): m/z = 560 [M+H]⁺
(8) 1-(2-Cyano-benzyl)-3-methyl-7-(2-butin-1-yl)-8-{(*S*)-[2-(tert.-butoxycarbonyl-amino)-propyl]-methylamino}-xanthin
   (Durchführung mit Kalium-tert.-butylat in Dimethylsulfoxid)
   R_{f}-Wert: 0.38 (Kieselgel, Cyclohexan/Essigester =1:1)
   Massenspektrum (ESI⁺): m/z = 520 [M+H]⁺

### Beispiel III

### 1-[(3-Methyl-isochinolin-1-yl)methyl]-3-methyl-7-(2-butin-1-yl)-8-[2-(tert.-butoxy-carbonylamino)-2-methyl-propylamino]-xanthin

Zu 810 mg 1-[(3-Methyl-isochinolin-1-yl)methyl]-3-methyl-7-(2-butin-1-yl)-8-(2-amino-2-methyl-propylamino)-xanthin und 0.33 ml Diisopropylethylamin in 20 ml Methanol werden unter Eiskühlung 405 mg Pyrokohlensäure-di-tert.-butylester gegeben. Das Reaktionsgemisch wird auf Raumtemperatur erwärmt und über Nacht gerührt.

Zur Aufarbeitung werden 40 ml Eiswasser zugegeben und der enstandene Niederschlag wird abgesaugt, mit Wasser, etwas Methanol und tert.-Butylmethylether gewaschen und im Exsikkator getrocknet.
Ausbeute: 810 mg (82 % der Theorie)
R_{f}-Wert: 0.75 (Kieselgel, Methylenchlorid/Methanol/konz. wässriges Ammoniak = 90:10:1)
Massenspektrum (ESI⁺): m/z = 560 [M+H]⁺

Analog Beispiel III werden folgende Verbindungen erhalten:
(1) 1-[(4-Methyl-chinazolin-2-yl)methyl]-3-methyl-7-(2-butin-1-yl)-8-[2-(tert.-butoxycarbonylamino)-2-methyl-propylamino]-xanthin
   R_{f}-Wert: 0.54 (Kieselgel, Methylenchlorid/Methanol/konz. wässriges Ammoniak = 90:10:1)
   Massenspektrum (ESI⁺): m/z = 561 [M+H]⁺
(2) 1-[(3-Methyl-isochinolin-1-yl)methyl]-3-methyl-7-(2-butin-1-yl)-8-[(*S*)-2-(tert.-butoxycarbonylamino)-propylamino]-xanthin
   R_{f}-Wert: 0.75 (Kieselgel, Essigester)
   Massenspektrum (ESI⁺): m/z = 546 [M+H]⁺
(3) 1-[(3-Methyl-isochinolin-1-yl)methyl]-3-methyl-7-(2-butin-1-yl)-8-[(*R*)-2-(tert.-butoxycarbonylamino)-propylamino]-xanthin
   Massenspektrum (ESI⁺): m/z = 546 [M+H]⁺
(4) 1-[(4-Methyl-chinazolin-2-yl)methyl]-3-methyl-7-(2-butin-1-yl)-8-[(*S*)-2-(tert.-butoxycarbonylamino)-propylamino]-xanthin
   R_{f}-Wert: 0.30 (Kieselgel, Essigester)
   Massenspektrum (ESI⁺): m/z = 547 [M+H]⁺
(5) 1-[(4-Methyl-chinazolin-2-yl)methyl]-3-methyl-7-(2-butin-1-yl)-8-[(*R*)-2-(tert.-butoxycarbonylamino)-propylamino]-xanthin
   R_{f}-Wert: 0.30 (Kieselgel, Essigester)
   Massenspektrum (ESI⁺): m/z = 547 [M+H]⁺
(6) 1-(2-Cyano-benzyl)-3-methyl-7-(2-butin-1-yl)-8-[(*S*)-2-(tert.-butoxycarbonylamino)-propylamino]-xanthin
   R_{f}-Wert: 0.60 (Kieselgel, Essigester/Petrolether = 7:3)
   Massenspektrum (ESI⁺): m/z = 506 [M+H]⁺

### Beispiel IV

### 1-[(3-Methyl-isochinolin-1-yl)methyl]-3-methyl-7-(2-butin-1-yl)-8-(2-amino-2-methyl-propylamino)-xanthin

Ein Gemisch aus 1.00 g 1-[(3-Methyl-isochinolin-1-yl)methyl]-3-methyl-7-(2-butin-1-yl)-8-brom-xanthin, 0.625 ml 1,2-Diamino-2-methylpropan und 500 mg Natriumcarbonat in 5 ml N-Methylpyrrolidon wird in der Mikrowelle für drei Minuten auf 200°C erhitzt. Das Reaktionsgemisch wird mit Wasser verrührt und mit Essigester extrahiert. Die vereinigten organischen Phasen werden mit Wasser gewaschen, über Magnesiumsulfat getrocknet und eingeengt.
Ausbeute: 830 mg (82 % der Theorie)
R_{f}-Wert: 0.43 (Kieselgel, Methylenchlorid/Methanol/konz. wässriges Ammoniak = 90:10:1)
Massenspektrum (ESI⁺): m/z = 460 [M+H]⁺

Analog Beispiel IV werden folgende Verbindungen erhalten:
(1) 1-[(4-Methyl-chinazolin-2-yl)methyl]-3-methyl-7-(2-butin-1-yl)-8-(2-amino-2-methyl-propylamino)-xanthin
   R_{f}-Wert: 0.39 (Kieselgel, Methylenchlorid/Methanol/konz. wässriges Ammoniak = 90:10:1)
   Massenspektrum (ESI⁺): m/z = 461 [M+H]⁺
(2) 1-[(3-Methyl-isochinolin-1-yl)methyl]-3-methyl-7-(2-butin-1-yl)-8-((*S*)-2-amino-propylamino)-xanthin
   R_{f}-Wert: 0.25 (Kieselgel, Methylenchlorid/Methanol/konz. wässriges Ammoniak = 90:10:0.1)
(3) 1-[(3-Methyl-isochinolin-1-yl)methyl]-3-methyl-7-(2-butin-1-yl)-8-((*R*)-2-amino-propylamino)-xanthin
   Massenspektrum (ESI⁺): m/z = 446 [M+H]⁺
(4) 1-[(4-Methyl-chinazolin-2-yl)methyl]-3-methyl-7-(2-butin-1-yl)-8-((*S*)-2-amino-propylamino)-xanthin
   R_{f}-Wert: 0.20 (Kieselgel, Methylenchlorid/Methanol/konz. wässriges Ammoniak = 90:10:0.1)
(5) 1-[(4-Methyl-chinazolin-2-yl)methyl]-3-methyl-7-(2-butin-1-yl)-8-((*R*)-2-amino-propylamino)-xanthin
(6) 1-[(3-Methyl-isochinolin-1-yl)methyl]-3-methyl-7-(2-butin-1-yl)-8-[(*R*)-2-(tert.-butoxycarbonylamino)-1-methyl-ethylamino]-xanthin
   (Durchführung in Gegenwart von Kaliumcarbonat in Dimethylsulfoxid bei 80°C)
   R_{f}-Wert: 0.60 (Kieselgel, Essigester)
   Massenspektrum (ESI⁺): m/z = 546 [M+H]⁺
(7) 1-[(3-Methyl-isochinolin-1-yl)methyl]-3-methyl-7-(2-butin-1-yl)-8-[(*R*)-2-(tert.-butoxycarbonylamino)-1-methyl-ethylamino]-xanthin
   (Durchführung in Gegenwart von N-Methylmorpholin in Dimethylsulfoxid bei 80°C)
   R_{f}-Wert: 0.60 (Kieselgel, Essigester)
   Massenspektrum (ESI⁺): m/z = 546 [M+H]⁺
(8) 1-(2-Cyano-benzyl)-3-methyl-7-(2-butin-1-yl)-8-((*S*)-2-amino-propylamino)-xanthin (Durchführung in Gegenwart von Kaliumcarbonat in N-Methylpyrrolidon bei 120°C)
   R_{f}-Wert: 0.25 (Kieselgel, Methylenchlorid/Methanol/konz. wässriges Ammoniak = 90:10:0.1)
(9) 1-[([1,5]Naphthyridin-2-yl)methyl]-3-methyl-7-(2-butin-1-yl)-8-{(*S*)-[2-(tert.-butoxycarbonylamino)-propyl]-methylamino}-xanthin
   (Durchführung in Gegenwart von Kaliumcarbonat in Dimethylsulfoxid bei 110°C)
   Massenspektrum (ESI⁺): m/z = 547 [M+H]⁺
(10) 1-[(Chinoxalin-6-yl)methyl]-3-methyl-7-(2-butin-1-yl)-8-{(*S*)-[2-(tert.-butoxycarbonylamino)-propyl]-methylamino}-xanthin
   (Durchführung in Gegenwart von Kaliumcarbonat in Dimethylsulfoxid bei 110°C)
   R_{f}-Wert: 0.55 (Kieselgel, Essigester)
   Massenspektrum (ESI⁺): m/z = 547 [M+H]⁺
(11) 1-[(3-Cyano-pyridin-2-yl)methyl]-3-methyl-7-(2-butin-1-yl)-8-{(S)-[2-(tert.-butoxycarbonylamino)-propyl]-methylamino}-xanthin
   (Durchführung in Gegenwart von Kaliumcarbonat in Dimethylsulfoxid bei 110°C)
   R_{f}-Wert: 0.65 (Kieselgel, Essigester)
   Massenspektrum (ESI⁺): m/z = 521 [M+H]⁺
(12) 3-Methyl-7-(2-butin-1-yl)-8-{(*S*)-[2-(tert.-butoxycarbonylamino)-propyl]-methylamino}-xanthin
   (Durchführung in Gegenwart von Kaliumcarbonat in Dimethylsulfoxid bei 120°C)
   R_{f}-Wert: 0.50 (Kieselgel, Essigester)
(13) 1-(2-Cyano-benzyl)-3-methyl-7-(2-butin-1-yl)-8-{[2-(tert.-butoxycarbonylamino)-2-methyl-propyl]-methylamino}-xanthin
   (Durchführung in Gegenwart von Kaliumcarbonat in N-Methylpyrrolidon bei 85°C)
   R_{f}-Wert: 0.70 (Kieselgel, Methylenchlorid/Methanol = 4:1)
   Massenspektrum (ESI⁺): m/z = 534 [M+H]⁺
(14) 1-[(3-Cyano-pyridin-2-yl)methyl]-3-methyl-7-(2-butin-1-yl)-8-{[2-(tert.-butoxy-carbonylamino)-2-methyl-propyl]-methylamino}-xanthin
   (Durchführung in Gegenwart von Kaliumcarbonat in N-Methylpyrrolidon bei 85°C)
   R_{f}-Wert: 0.30 (Kieselgel, Methylenchlorid/Methanol = 4:1)
   Massenspektrum (ESI⁺): m/z = 535 [M+H]⁺
(15) 1-(4-Cyano-benzyl)-3-methyl-7-(2-butin-1-yl)-8-{[2-(tert.-butoxycarbonylamino)-2-methyl-propyl]-methylamino}-xanthin
   (Durchführung in Gegenwart von Kaliumcarbonat in N-Methylpyrrolidon bei 85°C)
   R_{f}-Wert: 0.80 (Kieselgel, Methylenchlorid/Methanol = 9:1)
   Massenspektrum (ESI⁺): m/z = 534 [M+H]⁺
(16) 1-(3-Cyano-benzyl)-3-methyl-7-(2-butin-1-yl)-8-{[2-(tert.-butoxycarbonylamino)-2-methyl-propyl]-methylamino}-xanthin
   (Durchführung in Gegenwart von Kaliumcarbonat in N-Methylpyrrolidon bei 85°C)
   R_{f}-Wert: 0.85 (Kieselgel, Methylenchlorid/Methanol = 9:1)
   Massenspektrum (ESI⁺): m/z = 534 [M+H]⁺

### Beispiel V

### ((S)-1-Methyl-2-methylamino-ethyl)-carbaminsäure-tert.-butylester

Zu 1.73 g ((S)-1-Methyl-2-oxoethyl)-carbaminsäure-tert.-butylester in 20 ml Benzol werden unter Rühren bei Raumtemperatur 6.00 ml Methylamin-Lösung (2 M in Tetrahydrofuran) gegeben. Anschließend werden 2.50 g wasserfreies Natriumsulfat zugegeben und das Reaktionsgemisch wird über Nacht bei Raumtemperatur gerührt. Das Natriumsulfat wird abgesaugt und mit Benzol nachgewaschen. Das Filtrat wird eingeengt, in 20 ml Methanol aufgenommen und unter Rühren bei Raumtemperatur mit 397 mg Natriumcyanoborhydrid versetzt. Nach 2.5 Stunden wird das Reaktionsgemisch durch Zutropfen von ca. 20 ml 2 N Citronensäure angesäuert und mit tert.-Butylmethylether extrahiert. Die wässrige Phase wird mit 10 M Natronlauge alkalisch gestellt und mit einem Methylenchlorid/Methanol-Gemisch extrahiert. Die vereinigten organischen Phasen werden über Magnesiumsulfat getrocknet und eingeengt.
Ausbeute: 1.06 g (56 % der Theorie)
Massenspektrum (ESI⁺): m/z = 189 [M+H]⁺

### Beispiel VI

### 3-Brommethyl-4-cyano-isochinolin

Zu einer siedenden Mischung aus 400 mg 3-Methyl-4-cyano-isochinolin und 440 mg N-Bromsuccinimid in 30 ml Tetrachlorkohlenstoff werden 39 mg Azo-isobutyronitril gegeben. Das Reaktionsgemisch wird zwei Stunden unter Rückfluß gerührt. Nach Abkühlung auf Raumtemperatur wird der ausgefallene Niederschlag abgesaugt und mit Tetrachlorkohlenstoff gewaschen. Das Filtrat wird eingeengt und über eine Kieselgelsäule mit Methylenchlorid als Laufmittel chromatographiert.
Ausbeute: 350 mg (60 % der Theorie)
R_{f}-Wert: 0.40 (Kieselgel, Petrolether/Essigester = 7:3)
Massenspektrum (ESI⁺): m/z = 247, 249 [M+H]⁺

### Beispiel VII

### 3-Methyl-4-cyano-isochinolin

Ein Gemisch aus 3.90 g 3-Methyl-4-brom-isochinolin, 1.47 g Zinkcyanid und 2.03 g Tetrakis(triphenylphosphin)palladium in 70 ml N-Methylpyrrolidon wird 27 Stunden unter Argonatmosphäre bei 105°C gerührt. Nach Abkühlung auf Raumtemperatur wird das Reaktionsgemisch mit 200 ml Cyclohexan versetzt und unter Eiskühlung mit 150 ml konz. Ammoniak verrührt. Die wässrige Phase wird abgetrennt und mit Cyclohexan extrahiert. Die vereinigten organischen Phasen werden mit konz. Ammoniak und gesättigter Natriumchlorid-Lösung gewaschen, über Magnesiumsulfat getrocknet und eingeengt. Das Rohprodukt wird über eine Kieselgelsäule mit Petrolether/Essigester (8:2) als Laufmittel gereinigt.
Ausbeute: 1.90 g (64 % der Theorie)
R_{f}-Wert: 0.30 (Kieselgel, Petrolether/Essigester = 7:3)
Massenspektrum (ESI⁺): m/z = 169 [M+H]⁺

### Beispiel VIII

### (1,1-Dimethyl-2-methylamino-ethyl)-carbaminsäure-tert.-butylester

22.81 g [2-(Benzyl-methyl-amino)-1,1-dimethyl-ethyl]-carbaminsäure-tert.-butylester in 200 ml Ethanol werden in Gegenwart von 2.30 g Palladium auf Aktivkohle (10%) drei Stunden bei Raumtemperatur und einem Wasserstoffpartialdruck von 68 psi hydriert. Anschließend wird der Katalysator abfiltriert und das Filtrat eingeengt, wobei ein farbloses Öl zurückbleibt.
Ausbeute: 14.38 g (91 % der Theorie)
R_{f}-Wert: 0.55 (Kieselgel, Cyclohexan/Essigester = 2:1)
Massenspektrum (ESI⁺): m/z = 203 [M+H]⁺

### Beispiel IX

### [2-(Benzyl-methyl-amino)-1,1-dimethyl-ethyl]-carbaminsäure-tert.-butylester

Ein Gemisch aus 34.41 g N¹-Benzyl-N¹,2-dimethyl-propan-1,2-diamin in 280 ml Ethanol, 25.20 ml Triethylamin und 39.20 g Pyrokohlensäure-di-tert.-butylester wird zwei Stunden bei Raumtemperatur gerührt. Anschließend wird das Lösungsmittel abdestilliert und der Kolbenrückstand chromatographisch über Kieselgel mit Cyclohexan/Essigester (100:0 auf 66:34) als Laufmittel gereinigt.
Ausbeute: 22.81 g (44 % der Theorie)
R_{f}-Wert: 0.80 (Kieselgel, Cyclohexan/Essigester = 2:1)
Massenspektrum (ESI⁺): m/z = 293 [M+H]⁺

### Beispiel X

### N¹-Benzyl-N¹,2-dimethyl-propan-1,2-diamin

Analog der Vorschrift von K. Taniguchi et al., Chem. Pharm. Bull. 1995, 43, 71-77, werden 39.78 g *N*-Methyl-N-(2-methyl-2-nitro-propyl)benzylamin in 280 ml Ethanol in Gegenwart von 7.20 g Raney-Nickel drei Stunden bei Raumtemperatur und einem Wasserstoffpartialdruck von 53 psi hydriert. Anschließend wird der Katalysator abfiltriert und das Filtrat wird direkt weiter umgesetzt (siehe Beispiel IX).
R_{f}-Wert: 0.20 (Kieselgel, Cyclohexan/Essigester = 2:1)

### Beispiel XI

### N-Methyl-N-(2-methyl-2-nitro-propyl)benzylamin

Hergestellt aus 2-Nitropropan, N-Methylbenzylamin und 37 %iger Formaldehydlösung in Gegenwart von Natronlauge in Dioxan analog der Vorschrift von K. Taniguchi et al., Chem. Pharm. Bull. 1995, 43, 71-77.
R_{f}-Wert: 0.85 (Kieselgel, Cyclohexan/Essigester = 2:1)
Massenspektrum (ESI⁺): m/z = 223 [M+H]⁺

### Herstellung der Endverbindungen:

### Beispiel 1

### 1-[(3-Methyl-isochinolin-1-yl)methyl]-3-methyl-7-(2-butin-1-yl)-8-[(2-amino-ethyl)-methylamino]-xanthin

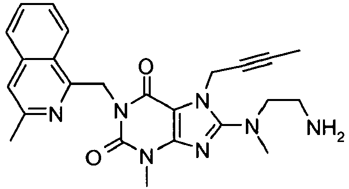

Ein Gemisch aus 250 mg 1-[(3-Methyl-isochinolin-1-yl)methyl]-3-methyl-7-(2-butin-1-yl)-8-brom-xanthin, 0.30 ml N-Methyl-ethylendiamin und 75 mg Natriumcarbonat in 3 ml Dimethylsulfoxid wird drei Stunden bei 70°C gerührt. Zur Aufarbeitung wird es mit Wasser versetzt und mit Methylenchorid extrahiert. Die vereinigten Extrakte werden über Magnesiumsulfat getrocknet und eingeengt. Der Kolbenrückstand wird über eine Kieselgelsäule mit Methylenchorid/Methanol (10:1 auf 2:1) als Laufmittel chromatographiert. Es werden 75 mg des Titelproduktes sowie 86 mg des isomeren Produktes 1-[(3-Methyl-isochinolin-1-yl)methyl]-3-methyl-7-(2-butin-1-yl)-8-(2-methyl-amino-ethylamino)-xanthin erhalten.
Ausbeute: 75 mg (30 % der Theorie)
Massenspektrum (ESI⁺): m/z = 447 [M+H]⁺

Analog Beispiel 1 werden folgende Verbindungen erhalten:

### (1) 1-[(4-Methyl-chinazolin-2-yl)methyl]-3-methyl-7-(2-butin-1-yl)-8-[(2-amino-ethyl)-methylamino]-xanthin

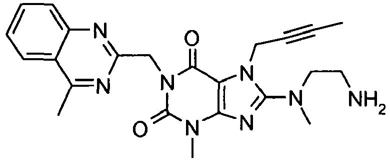

Massenspektrum (ESI⁺): m/z = 447 [M+H]⁺

### Beispiel 2

### 1-[(3-Methyl-isochinolin-1-yl)methyl]-3-methyl-7-(2-butin-1-yl)-8-[(2-amino-2-methylpropyl)-methylamino]-xanthin

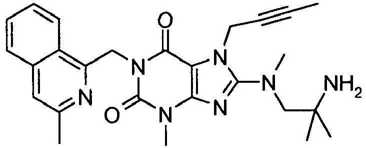

Eine Lösung aus 610 mg 1-[(3-Methyl-isochinolin-1-yl)methyl]-3-methyl-7-(2-butin-1-yl)-8-{[2-(tert.-butoxycarbonylamino)-2-methyl-propyl]-methylamino}-xanthin in 10 ml Methylenchorid wird mit 2.20 ml isopropanolischer Salzsäure [5-6 M] versetzt und über Nacht bei Raumtemperatur gerührt. Das Reaktionsgemisch wird mit Methylenchlorid verdünnt und mit Wasser extrahiert. Die vereinigten wässrigen Phasen werden mit gesättigter Natriumhydrogencarbonat-Lösung alkalisch gestellt und mit Methylenchlorid extrahiert. Die vereinigten organischen Phasen werden über Magnesiumsulfat getrocknet und eingeengt. Das Rohprodukt wird mit tert.-Butyl-methyl-ether verrührt, abgesaugt, mit wenig tert.-Butylmethylether gewaschen und in der Trockenpistole bei 60°C getrocknet.
Ausbeute: 440 mg (87 % der Theorie)
R_{f}-Wert: 0.46 (Kieselgel, Methylenchlorid/Methanol/konz. wässriges Ammoniak = 90:10:1)
Massenspektrum (ESI⁺): m/z = 474 [M+H]⁺

Analog Beispiel 2 werden folgende Verbindungen erhalten:
(1) 1-[(4-Methyl-chinazolin-2-yl)methyl]-3-methyl-7-(2-butin-1-yl)-8-[(2-amino-2-methyl-propyl)-methylamino]-xanthin 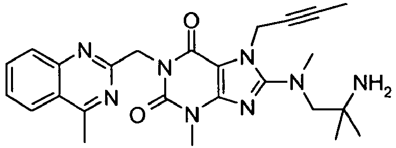 R_{f}-Wert: 0.57 (Kieselgel, Methylenchlorid/Methanol/konz. wässriges Ammoniak = 90:10:1)
   Massenspektrum (ESI⁺): m/z = 475 [M+H]⁺
(2) 1-[(3-Methyl-isochinolin-1-yl)methyl]-3-methyl-7-(2-butin-1-yl)-8-[(*S*)-(2-amino-propyl)-methylamino]-xanthin 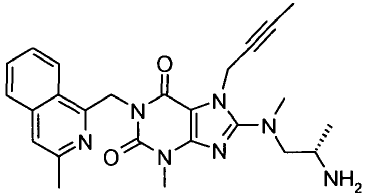 R_{f}-Wert: 0.45 (Kieselgel, Methylenchlorid/Methanol/konz. wässriges Ammoniak = 90:10:0.1)
   Massenspektrum (ESI⁺): m/z = 460 [M+H]⁺
(3) 1-[(3-Methyl-isochinolin-1-yl)methyl]-3-methyl-7-(2-butin-1-yl)-8-[(*R*)-(2-amino-propyl)-methylamino]-xanthin 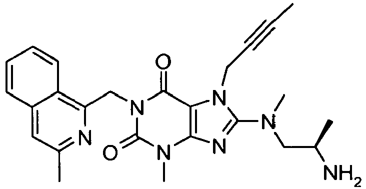 Massenspektrum (ESI⁺): m/z = 460 [M+H]⁺
(4) 1-[(4-Methyl-chinazolin-2-yl)methyl]-3-methyl-7-(2-butin-1-yl)-8-[(*S*)-(2-amino-propyl)-methylamino]-xanthin 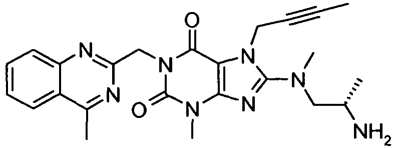 R_{f}-Wert: 0.40 (Kieselgel, Methylenchlorid/Methanol/konz. wässriges Ammoniak = 90:10:0.1)
   Massenspektrum (ESI⁺): m/z = 461 [M+H]⁺
(5) 1-[(4-Methyl-chinazolin-2-yl)methyl]-3-methyl-7-(2-butin-1-yl)-8-[(*R*)-(2-amino-propyl)-methylamino]-xanthin 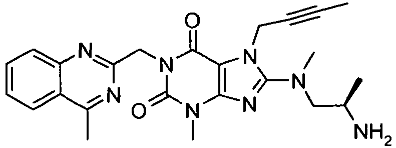 (BOC-Spaltung erfolgt mit Trifluoressigsäure in Methylenchlorid)
   R_{f}-Wert: 0.40 (Kieselgel, Methylenchlorid/Methanol/konz. wässriges Ammoniak = 90:10:0.1)
   Massenspektrum (ESI⁺): m/z = 461 [M+H]⁺
(6) 1-[(3-Methyl-isochinolin-1-yl)methyl]-3-methyl-7-(2-butin-1-yl)-8-[(*R*)-(2-amino-1-methyl-ethyl)-methylamino]-]-xanthin 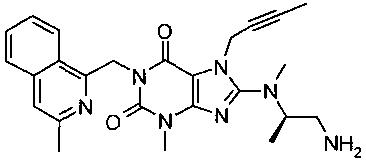 (BOC-Spaltung erfolgt mit Trifluoressigsäure in Methylenchlorid)
   R_{f}-Wert: 0.40 (Kieselgel, Methylenchlorid/Methanol/konz. wässriges Ammoniak = 90:10:0.1)
   Massenspektrum (ESI⁺): m/z = 460 [M+H]⁺
(7) 1-[(3-Methyl-isochinolin-1-yl)methyl]-3-methyl-7-(2-butin-1-yl)-8-[(*S*)-(2-amino-1-methyl-ethyl)-methylamino]-]-xanthin 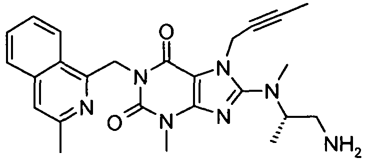 (BOC-Spaltung erfolgt mit Trifluoressigsäure in Methylenchlorid)
   Massenspektrum (ESI⁺): m/z = 460 [M+H]⁺
(9) 1-[([1,5]Naphthyridin-2-yl)methyl]-3-methyl-7-(2-butin-1-yl)-8-[(*S*)-(2-amino-propyl)-methylamino]-xanthin 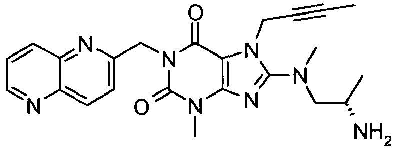 R_{f}-Wert: 0.47 (Kieselgel, Methylenchlorid/Methanol/konz. wässriges Ammoniak = 90:10:1)
   Massenspektrum (ESI⁺): m/z = 447 [M+H]⁺
(10) 1-[(Chinoxalin-6-yl)methyl]-3-methyl-7-(2-butin-1-yl)-8-[(*S*)-(2-amino-propyl)-methylamino]-xanthin 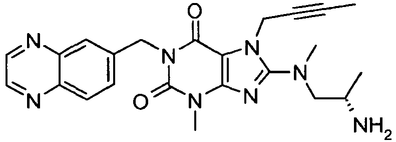 R_{f}-Wert: 0.50 (Kieselgel, Methylenchlorid/Methanol/konz. wässriges Ammoniak = 90:10:1)
   Massenspektrum (ESI⁺): m/z = 447 [M+H]⁺
(11) 1-[(3-Cyano-pyridin-2-yl)methyl]-3-methyl-7-(2-butin-1-yl)-8-[(*S*)-(2-amino-propyl)-methylamino]-xanthin 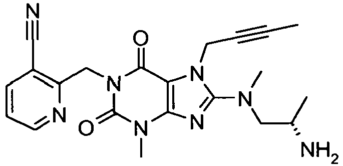 R_{f}-Wert: 0.55 (Kieselgel, Methylenchlorid/Methanol/konz. wässriges Ammoniak = 90:10:0.1)
   Massenspektrum (ESI⁺): m/z = 421 [M+H]⁺
(12) 1-[(4-Cyano-isochinolin-3-yl)methyl]-3-methyl-7-(2-butin-1-yl)-8-[(*S*)-(2-amino-propyl)-methylamino]-xanthin 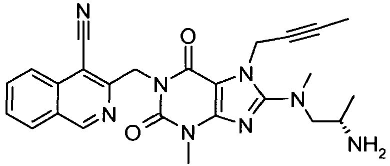 (BOC-Spaltung erfolgt mit Trifluoressigsäure in Methylenchlorid)
   R_{f}-Wert: 0.45 (Kieselgel, Methylenchlorid/Methanol/konz. wässriges Ammoniak = 90:10:0.1)
   Massenspektrum (ESI⁺): m/z = 471 [M+H]⁺
(14) 1-[(3-Cyano-pyridin-2-yl)methyl]-3-methyl-7-(2-butin-1-yl)-8-[(2-amino-2-methylpropyl)-methylamino]-xanthin 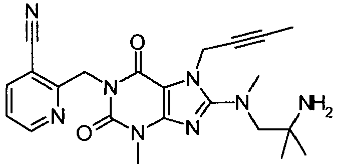 (BOC-Spaltung erfolgt mit Trifluoressigsäure in Methylenchlorid)
   Massenspektrum (ESI⁺): m/z = 435 [M+H]⁺
   R_{f}-Wert: 0.50 (Reversed Phase DC-Fertigplatte (E. Merck), Acetonitril/Wasser/ Trifluoressigsäure = 50:50:1)

Analog den vorstehenden Beispielen und anderen literaturbekannten Verfahren können auch die folgenden Verbindungen erhalten werden:

| Bsp. | Struktur | Bsp. | Struktur |
|---|---|---|---|
| (1) | 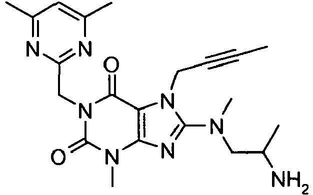 | (2) | 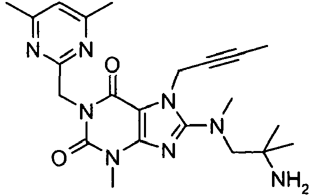 |
| (3) | 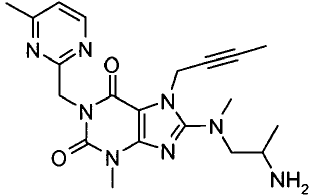 | (4) | 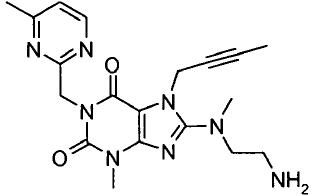 |
| (5) | 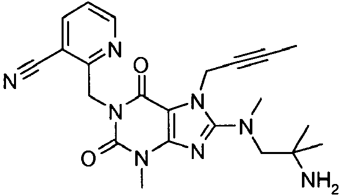 | (6) | 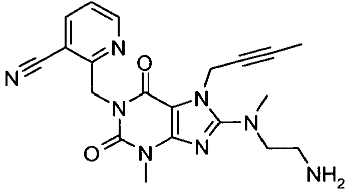 |
| (11) | 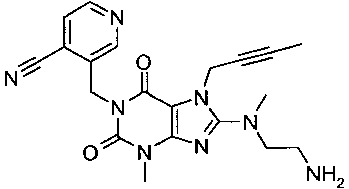 | (12) | 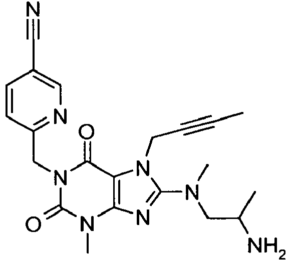 |
| (13) | 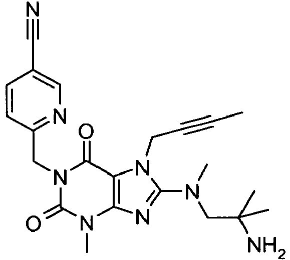 | | |
| (19) | 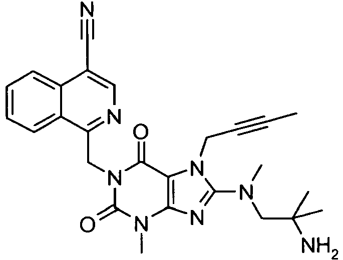 | (20) | 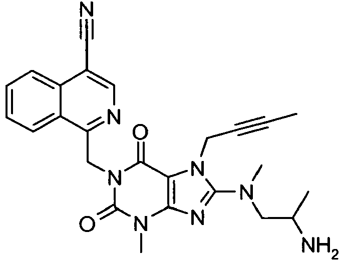 |
| (21) | 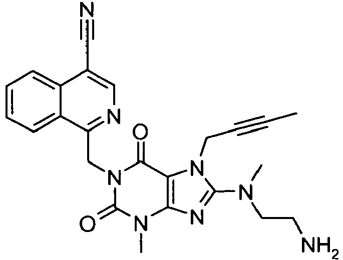 | | |
| (25) | 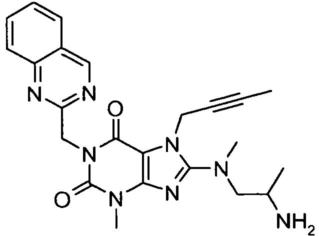 | (26) | 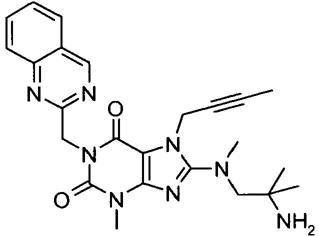 |
| (27) | 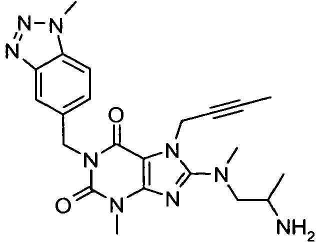 | (28) | 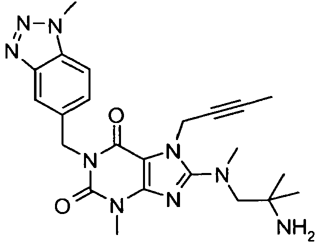 |
| (29) | 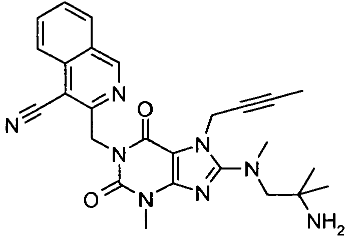 | (30) | 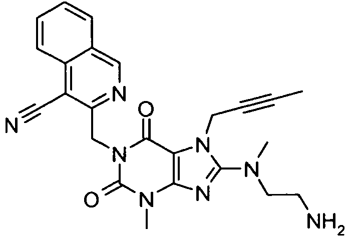 |
| (31) | 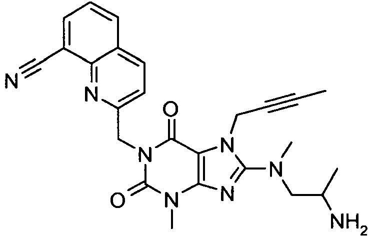 | (32) | 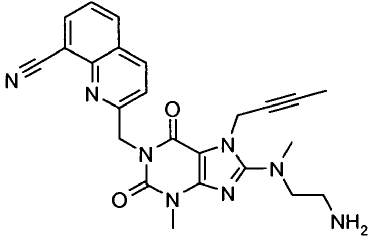 |
| (33) | 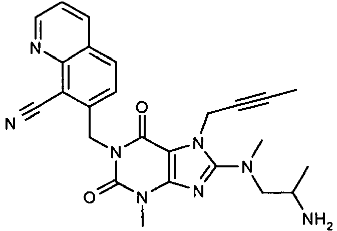 | (34) | 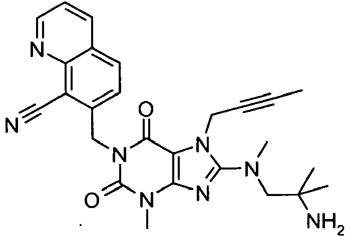 |
| (35) | 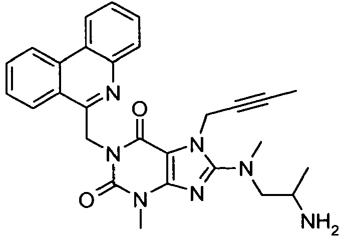 | (36) | 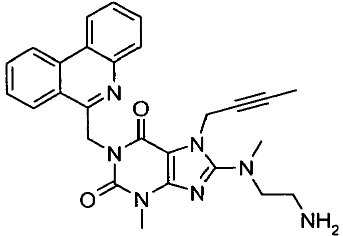 |
| (37) | 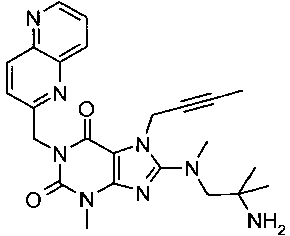 | (38) | 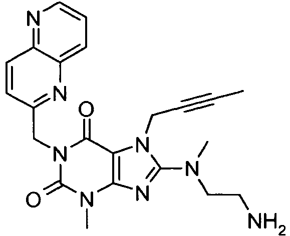 |
| (39) | 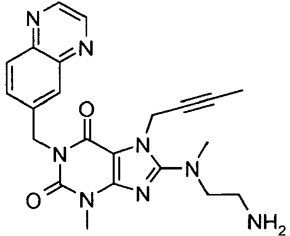 | (40) | 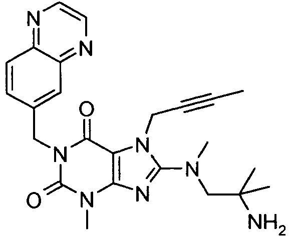 |
| (41) | 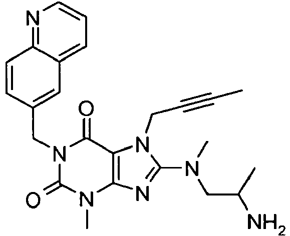 | (42) | 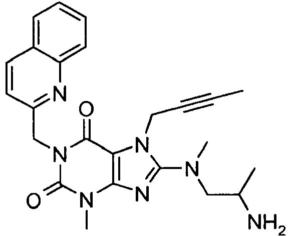 |
| (43) | 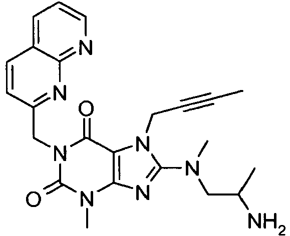 | (44) | 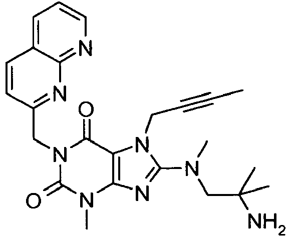 |
| (45) | 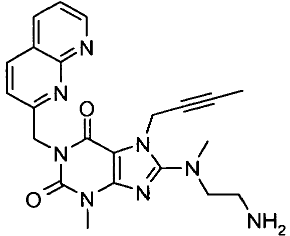 | (46) | 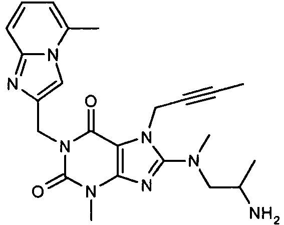 |
| (47) | 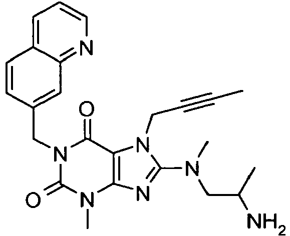 | (48) | 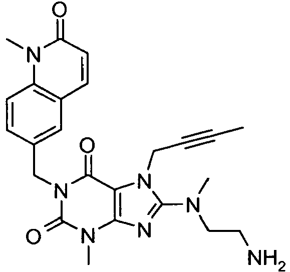 |
| (49) | 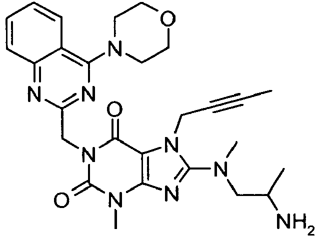 | (50) | 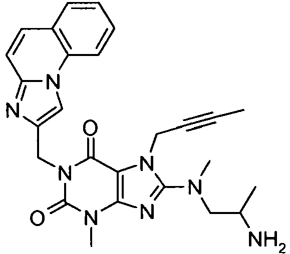 |
| (51) | 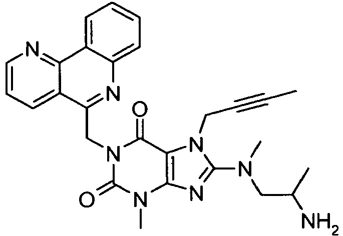 | (52) | 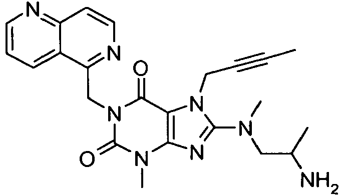 |
| (53) | 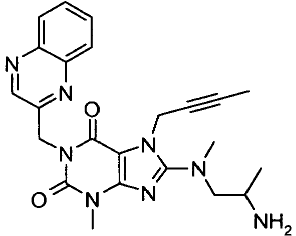 | (54) | 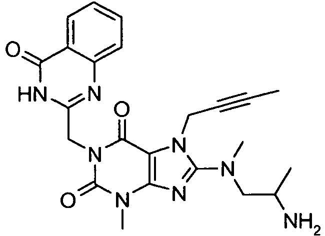 |
| (55) | 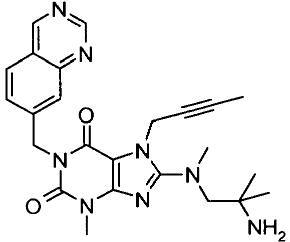 | (56) | 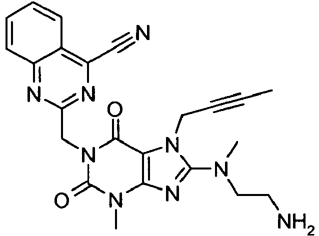 |
| (57) | 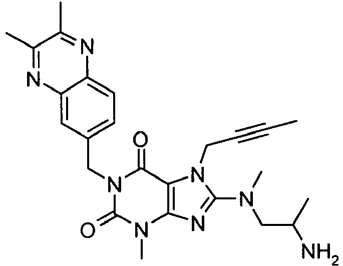 | (58) | 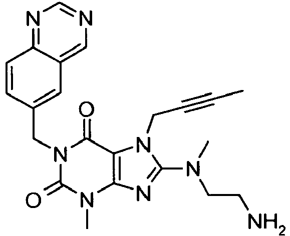 |
| | | (60) | 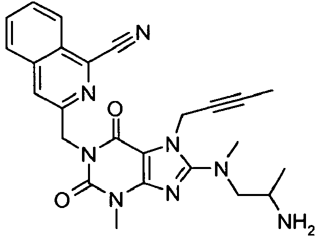 |
| (61) | 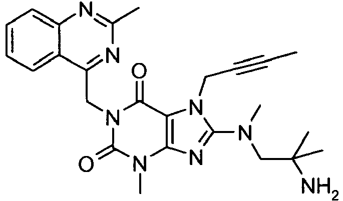 | (62) | 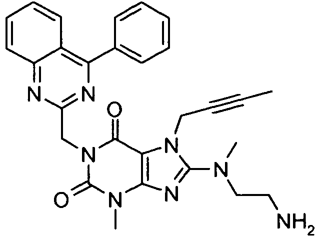 |
| (63) | 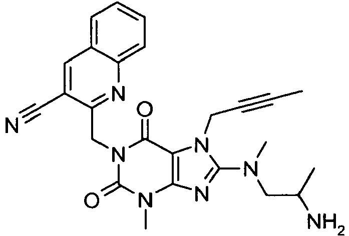 | (64) | 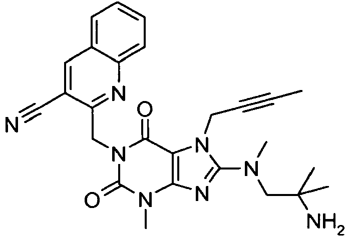 |
| (65) | 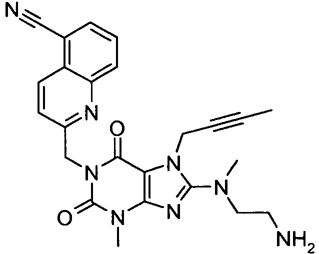 | (66) | 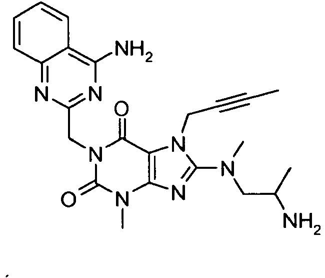 |
| (67) | 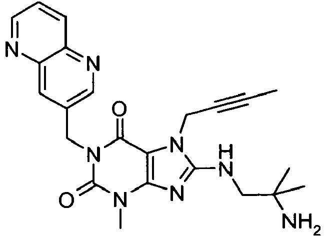 | (68) | 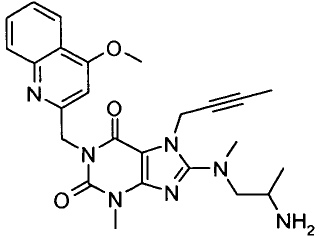 |

### Beispiel 3

### Dragées mit 75 mg Wirksubstanz

| 1 | Dragéekern enthält: | |
|---|---|---|
| | Wirksubstanz | 75,0 mg |
| | Calciumphosphat | 93,0 mg |
| | Maisstärke | 35,5 mg |
| | Polyvinylpyrrolidon | 10,0 mg |
| | Hydroxypropylmethylcellulose | 15,0 mg |
| | Magnesiumstearat | 1,5 mg |
| | | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.
Kerngewicht: 230 mg
Stempel: 9 mm, gewölbt

Die so hergestellten Dragéekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 4

### Tabletten mit 100 mg Wirksubstanz

| Zusammensetzung: | | |
|---|---|---|
| 1 | Tablette enthält: | |
| | Wirksubstanz | 100,0 mg |
| | Milchzucker | 80,0 mg |
| | Maisstärke | 34,0 mg |
| | Polyvinylpyrrolidon | 4,0 mg |
| | Magnesiumstearat | 2,0 mg |
| | | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.
Tablettengewicht: 220 mg
Durchmesser: 10 mm, biplan mit beidseitiger Facette und einseitiger Teilkerbe.

### Beispiel 5

### Tabletten mit 150 mg Wirksubstanz

| Zusammensetzung: | | |
|---|---|---|
| 1 | Tablette enthält: | |
| | Wirksubstanz | 150,0 mg |
| | Milchzucker pulv. | 89,0 mg |
| | Maisstärke | 40,0 mg |
| | Kolloide Kieselgelsäure | 10,0 mg |
| | Polyvinylpyrrolidon | 10,0 mg |
| | Magnesiumstearat | 1,0 mg |
| | | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 6

### Hartgelatine-Kapseln mit 150 mg Wirksubstanz

| 1 | Kapsel enthält: | |
|---|---|---|
| | Wirkstoff | 150,0 mg |
| | Maisstärke getr. | ca. 180,0 mg |
| | Milchzucker pulv. | ca. 87,0 mg |
| | Magnesiumstearat | 3,0 mg |
| | | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 7

### Suppositorien mit 150 mg Wirksubstanz

| 1 | Zäpfchen enthält: | |
|---|---|---|
| | Wirkstoff | 150,0 mg |
| | Polyethylenglykol 1500 | 550,0 mg |
| | Polyethylenglykol 6000 | 460,0 mg |
| | Polyoxyethylensorbitanmonostearat | 840,0 mg |
| | | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 8

### Suspension mit 50 mg Wirksubstanz

| 100 ml Suspension enthalten: | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

### Beispiel 9

### Ampullen mit 10 mg Wirksubstanz

| Zusammensetzung: | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 10

### Ampullen mit 50 mg Wirksubstanz

| Zusammensetzung: | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 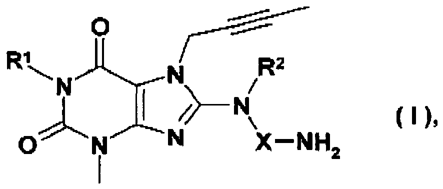 in denen
R¹ eine Heteroarylmethyl- oder Heteroarylethylgruppe,
eine Heteroarylcarbonylmethylgruppe oder
eine Arylprop-2-enyl- oder Heteroarylprop-2-enylgruppe, in denen die Propenylkette durch 1 bis 4 Fluoratome oder eine Cyan-, C₁₋₃-Alkyloxy-carbonyl- oder Nitrogruppe substituiert sein kann,
R² eine C₁₋₄-Alkylgruppe, welche geredkettig oder verzweigt sein kann, und
X eine -CH₂CH₂-Gruppe, welche gegebenenfalls durch ein oder zwei C₁₋₃-Alkylgruppen, die gleich oder verschieden sein können, substituiert sein kann, bedeuten,
wobei, soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische, deren Prodrugs und deren Salze.

2. Verbindungen der allgemeinen Formel I gemäß Anspruch 1, in denen
R¹ wie in Anspruch 1 erwähnt definiert ist,
R² eine Methyl- oder Ethylgruppe und
X eine -CH₂CH₂-Gruppe, welche gegebenenfalls durch ein oder zwei Methyl- oder Ethylgruppen substituiert sein kann, wobei die Substituenten gleich oder verschieden sein können, bedeuten
deren Tautomere, Enantiomere, Diastereomere, deren Gemische und deren Salze.

3. Verbindungen der allgemeinen Formel I gemäß Anspruch 2, in denen
R¹ eine Phenylprop-2-enyl-, Pyridinylmethyl-, Pyrimidinylmethyl-, Chinolinylmethyl-, Chinolinonylmethyl-, Imidazochinolinylmethyl-, Isochinollnylmethyl-, Chinazolinylmethyl-, Chinazolinonylmethyl-, Chinoxalinylmethyl-, Phenanthridinylmethyl-, Naphthyridinylmethyl-, Benzonaphthiridinylmethyl-, Imidazopyridinylmethyl- oder Benzotriazolyfmethylgruppe, die jeweils durch ein oder zwei Fluor-, Chlor-, Bromatome oder ein oder zwei Cyan-, Nitro-, Amino-, C₁₋₃-Alkyl-, C₁₋₃-Alkyloxy-, Phenyl-und Morpholinylgruppen substituiert sein können, wobei die Substituenten gleich oder verschieden sind,
R² eine Methylgruppe und
X eine -CH₂CH₂-Gruppe, welche gegebenenfalls durch ein oder zwei Methylgruppen substituiert sein kann, bedeuten,
deren Tautomere, Enantiomere, Diastereomere, deren Gemische und deren Salze.

4. Verbindungen der allgemeinen Formel I gemäß Anspruch 3, in denen
R¹ eine Pyridinylmethyl-, Pyrimidinylmethyl-, Chinolinylmethyl-, Isochinolinylmethyl-, Chinazolinylmethyl-, Chinoxalinylmethyl-, Naphthyridinylmethyl- oder Naphthylmethylgruppe, welche jeweils mit ein oder zwei Cyan- oder Methylgruppen substituiert sein können,
R² eine Methylgruppe und
X eine -CH(CH₃)-CH₂-Gruppe, eine -CH₂-CH(CH₃)-Gruppe oder eine -CH₂-C(CH₃)₂-Gruppe bedeuten,
deren Tautomere, Enantiomere, Diastereomere, deren Gemische und deren Salze.

5. Verbindungen der allgemeinen Formel I gemäß Anspruch 4, in denen
R¹ eine Pyridinylmethyl-, Isochinolinylmethyl-, Chinazolinylmethyl-, Chinoxalinylmethyl- oder Naphthyridinylmethylgruppe, welche jeweils mit einer Cyan-oder Methylgruppe substituiert sein können,
R² eine Methylgruppe und
X eine -CH(CH₃)-CH₂-Gruppe, eine -CH₂-CH(CH₃)-Gruppe oder eine -CH₂-C(CH₃)₂-Gruppe, wobei jeweils das rechts stehende Kohlenstoffatom mit der endständigen Aminogruppe verknüpft ist, bedeuten,
deren Tautomere, Enantiomere, Diastereomere, deren Gemische und deren Salze.

6. Folgende Verbindungen gemäß Anspruch 1:
(a) 1-[(3-Methyl-isochinolin-1-yl)methyl]-3-methyl-7 -(2-butin-1-yl)-8-[(2-aminoethyl)-methylamino]-xanthin
(b) 1-[(4-Methyl-chinazolin-2-yl)methyl]-3-methyl-7-(2-butin-1-yl)-8-[(2-amino-ethyl)-methylamino]-xanthin
(c) 1-[(3-Methyl-isochinolin-1-yl)methyl]-3-methyl-7-(2-butin-1-yl)-8-[(2-amino-2-methyl-propyl)-methylamino]-xanthin
(d) 1-[(4-Methyl-chinazolin-2-yl)methyl]-3-methyl-7-(2-butin-1-yl)-8-[(2-amino-2-methyl-propyl)-methylamino]-xanthin
(e) 1-[(3-Methyl-Isochinolin-1-yl)methyl]-3-methyl-7-(2-butin-1-yl)-8-[(S)-(2-amino-propyl)-methylamino]-xanthin
(f) 1-[(3-Methyl-isochinolin-1-yl)methyl]-3-methyl-7-(2-butin-1-yl)-8-[(R)-(2-amino-propyl)-methylamino]-xanthin
(g) 1-[(4-Methyl-chinazolin-2-yl)methyl]-3-methyl-7-(2-butin-1-yl)-8-[(S)-(2-amino-propyl)-methylamino]-xanthin
(h) 1-[(4-Methyl-chinazolin-2-yl)methyl]-3-methyl-7-(2-butin-1-yl)-8-[(R)-(2-amino-propyl)-methylamino]-xanthin
(i) 1-[(3-Methyl-isochinolin-1-yl)methyl]-3-methyl-7-(2-butin-1-yl)-8-[(R)-(2-amino-1-methyl-ethyl)-methylamino]-]-xanthin
(j) 1-[(3-Methyl-isochinolin-1-yl)methyl]-3-methyl-7-(2-butin-1-yl)-8-[(S)-(2-amino-1-methyl-ethyl)-methylaminol-1-xanthin
(k) 1-(2-Cyano-benxyl)-3-methyl-7-(2-butin-1-yl)-8-[(S)-(2-amino-propyl)-methyl-amino]-xanthin
(l) 1-[([1,5]Naphthyridin-2-yl)methyl]-3-methyl-7-(2-butin-1-yl)-8-[(S)-(2-amino-propyl)-methylamino]-xanthin
(m) 1-[(Chinoxalin-6-yl)methylj-3-methyl-7-(2-butin-1-yl)-8-[(S)-(2-amino-propyl)-methylamino]-xanthin
(n) 1-[(3-Cyano-pyridin-2-yl)methyl]-3-methyl-7-(2-butin-1-yl)-8-[(S)-(2-amino-propyl)-methylamino]-xanthin
(o) 1-[(4-Cyano-isochinolin-3-yl)methyl]-3-methyl-7-(2-butin-1-yl)-8-[(S)-(2-amino-propyl)-methylamino]-xanthin
(q) 1-[(3-Cyano-pyridin-2-yl)methyl]-3-methyl-7-(2-butin-1-yl)-8-[(2-amino-2-methylpropyl)-methylamino]-xanthin
sowie deren Salze.

7. Physiologisch verträgliche Salze der Verbindungen nach einem der Ansprüche 1 bis 6 mit anorganischen oder organischen Säuren oder Basen.

8. Arzneimittel, enthaltend eine Verbindung nach einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

10. Verfahren zur Herstellung eines Arzneimittels gemäß Anspruch 8, **dadurch gekennzeichnet, daß** auf nichtchemischen Weg eine Verbindung nach mindestens einem der Ansprüche 1 bis 7 in einen oder mehrere inerte Trägerstoffe und/oder Verdünnungsmittel eingearbeitet wird.

11. Verfahren zur Herstellung der Verbindungen der allgemeinen Formel 1 gemäß den Ansprüchen 1 bis 7, **dadurch gekennzeichnet, daß**
a) eine Verbindung der allgemeinen Formel 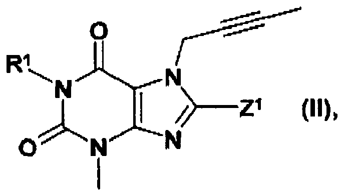 in der
R¹ wie in einem der Ansprüche 1 bis 6 erwähnt definiert ist und
Z¹ eine Austrittsgruppe wie ein Halogenatom, eine substituierte Hydroxy-, Mercapto-, Sulfinyl-, Sulfonyl- oder Sulfonyloxygruppe darstellt,
mit einer Verbindung der allgemeinen Formel 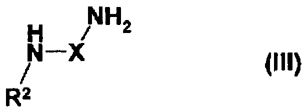 oder 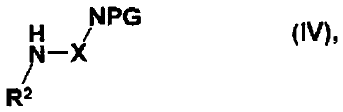 worin R² und X wie in einem der Ansprüche 1 bis 6 erwähnt definiert sind und NPG eine geschützte oder maskierte Aminofunktionalität darstellt, Derivaten oder Salzen davon, umgesetzt wird, oder
b) eine Verbindung der allgemeinen Formel 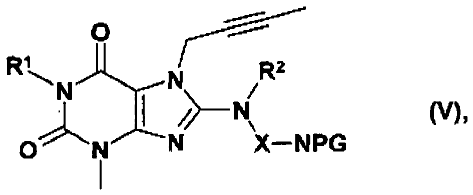
in der R¹, R² und X wie in einem der Ansprüche 1 bis 6 erwähnt definiert sind und NPG eine geschützte oder maskierte Aminofunktionalität darstellt, entschützt wird,
und/oder
anschließend gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.

## Claims

1. Compounds of general formula 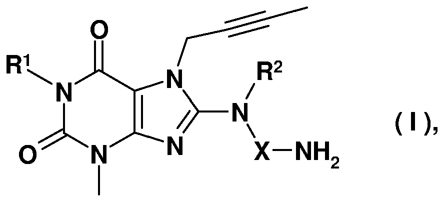 wherein
R¹ denotes a heteroarylmethyl or heteroarylethyl group,
a heteroarylcarbonylmethyl group or
an arylprop-2-enyl or heteroarylprop-2-enyl group, wherein the propenyl chain may be substituted by 1 to 4 fluorine atoms or a cyano, C₁₋₃-alkyloxy-carbonyl or nitro group,
R² denotes a C₁₋₄-alkyl group, which may be straight-chain or branched, and
X denotes a -CH₂CH₂ group, which may optionally be substituted by one or two C₁₋₃-alkyl groups, which may be identical or different,
wherein, unless otherwise stated, the above-mentioned alkyl, alkenyl and alkynyl groups may be straight-chain or branched,
the tautomers, enantiomers, diastereomers, the mixtures thereof, the prodrugs thereof and the salts thereof.

2. Compounds of general formula I according to claim 1, wherein
R¹ is defined as in claim 1,
R² denotes a methyl or ethyl group and
X denotes a -CH₂CH₂ group, which may optionally be substituted by one or two methyl or ethyl groups, wherein the substituents may be identical or different,
the tautomers, enantiomers, diastereomers, the mixtures thereof and the salts thereof.

3. Compounds of general formula I according to claim 2, wherein
R¹ denotes a phenylprop-2-enyl, pyridinylmethyl, pyrimidinylmethyl, quinolinylmethyl, quinolinonylmethyl, imidazoquinolinylmethyl, isoquinolinylmethyl, quinazolinylmethyl, quinazolinonylmethyl, quinoxalinylmethyl, phenanthridinylmethyl, naphthyridinylmethyl, benzonaphthiridinylmethyl, imidazopyridinylmethyl or benzo-triazolylmethyl group which may be substituted in each case by one or two fluorine, chlorine, bromine atoms or one or two cyano, nitro, amino, C₁₋₃-alkyl, C₁₋₃-alkyloxy, phenyl and morpholinyl groups, wherein the substituents are identical or different,
R² denotes a methyl group and
X denotes a -CH₂CH₂ group, which may optionally be substituted by one or two methyl groups,
the tautomers, enantiomers, diastereomers, the mixtures thereof and the salts thereof.

4. Compounds of general formula I according to claim 3, wherein
R¹ denotes a pyridinylmethyl, pyrimidinylmethyl, quinolinylmethyl, isoquinolinylmethyl, quinazolinylmethyl, quinoxalinylmethyl, naphthyridinylmethyl or naphthylmethyl group, which may be substituted in each case by one or two cyano or methyl groups,
R² denotes a methyl group and
X denotes a -CH(CH₃)-CH₂ group, a -CH₂-CH(CH₃) group or a -CH₂-C(CH₃)₂ group,
the tautomers, enantiomers, diastereomers, the mixtures thereof and the salts thereof.

5. Compounds of general formula I according to claim 4, wherein
R¹ denotes a pyridinylmethyl, isoquinolinylmethyl, quinazolinylmethyl, quinoxalinylmethyl or naphthyridinylmethyl group, each of which may be substituted by a cyano or methyl group,
R² denotes a methyl group and
X denotes a -CH(CH₃)-CH₂ group, a -CH₂-CH(CH₃) group or a -CH₂-C(CH₃)₂ group, wherein in each case the carbon atom on the right is linked to the terminal amino group,
the tautomers, enantiomers, diastereomers, the mixtures thereof and the salts thereof.

6. The following compounds according to claim 1:
(a) 1-[(3-methyl-isoquinolin-1-yl)methyl]-3-methyl-7-(2-butyn-1-yl)-8-[(2-aminoethyl)-methylamino]-xanthine
(b) 1-[(4-methyl-quinazolin-2-yl)methyl]-3-methyl-7-(2-butyn-1-yl)-8-[(2-aminoethyl)-methylamino]-xanthine
(c) 1-[(3-methyl-isoquinolin-1-yl)methyl]-3-methyl-7-(2-butyn-1-yl)-8-[(2-amino-2-methyl-propyl)-methylamino]-xanthine
(d) 1-[(4-methyl-quinazolin-2-yl)methyl]-3-methyl-7-(2-butyn-1-yl)-8-[(2-amino-2-methyl-propyl)-methylamino]-xanthine
(e) 1-[(3-methyl-isoquinolin-1-yl)methyl]-3-methyl-7-(2-butyn-1-yl)-8-[(S)-(2-aminopropyl)-methylamino]-xanthine
(f) 1-[(3-methyl-isoquinolin-1-yl)methyl]-3-methyl-7-(2-butyn-1-yl)-8-[(R)-(2-aminopropyl)-methylamino]-xanthine
(g) 1-[(4-methyl-quinazolin-2-yl)methyl]-3-methyl-7-(2-butyn-1-yl)-8-[(S)-(2-aminopropyl)-methylamino]-xanthine
(h) 1-[(4-methyl-quinazolin-2-yl)methyl]-3-methyl-7-(2-butyn-1-yl)-8-[(R)-(2-aminopropyl)-methylamino]-xanthine
(i) 1-[(3-methyl-isoquinolin-1-yl)methyl]-3-methyl-7-(2-butyn-1-yl)-8-[(R)-(2-amino-1-methyl-ethyl)-methylamino]-]-xanthine
(j) 1-[(3-methyl-isoquinolin-1-yl)methyl]-3-methyl-7-(2-butyn-1-yl)-8-[(S)-(2-amino-1-methyl-ethyl)-methylamino]-]-xanthine
(k) 1-(2-cyano-benzyl)-3-methyl-7-(2-butyn-1-yl)-8-[(S)-(2-amino-propyl)-methyl-amino]-xanthine
(l) 1-[([1,5]naphthyridin-2-yl)methyl]-3-methyl-7-(2-butyn-1-yl)-8-[(S)-(2-aminopropyl)-methylamino]-xanthine
(m) 1-[(quinoxalin-6-yl)methyl]-3-methyl-7-(2-butyn-1-yl)-8-[(S)-(2-amino-propyl)-methylamino]-xanthine
(n) 1-[(3-cyano-pyridin-2-yl)methyl]-3-methyl-7-(2-butyn-1-yl)-8-[(S)-(2-aminopropyl)-methylamino]-xanthine
(o) 1-[(4-cyano-isoquinolin-3-yl)methyl]-3-methyl-7-(2-butyn-1-yl)-8-[(S)-(2-aminopropyl)-methylamino]-xanthine
(q) 1-[(3-cyano-pyridin-2-yl)methyl]-3-methyl-7-(2-butyn-1-yl)-8-[(2-amino-2-methylpropyl)-methylamino]-xanthine
and the salts thereof.

7. Physiologically acceptable salts of the compounds according to one of claims 1 to 6 with inorganic or organic acids or bases.

8. Medicament, containing a compound according to one of claims 1 to 6 or a physiologically acceptable salt according to claim 7 optionally together with one or more inert carriers and/or diluents.

9. Use of a compound according to at least one of claims 1 to 7 for preparing a medicament which is suitable for the treatment of type I and type II diabetes mellitus, arthritis, obesity, allograft transplantation and osteoporosis caused by calcitonin.

10. Process for preparing a medicament according to claim 8, **characterised in that** a compound according to at least one of claims 1 to 7 is incorporated in one or more inert carriers and/or diluents by a non-chemical method.

11. Process for preparing the compounds of general formula I according to claims 1 to 7, **characterised in that**
a) a compound of general formula 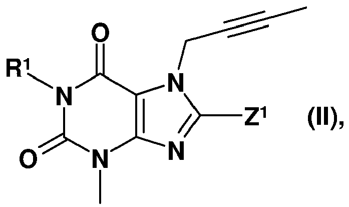 wherein
R¹ is defined as in one of claims 1 to 6 and
Z¹ denotes a leaving group such as a halogen atom, a substituted hydroxy, mercapto, sulphinyl, sulphonyl or sulphonyloxy group,
is reacted with a compound of general formula 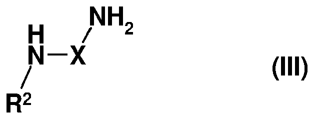 or 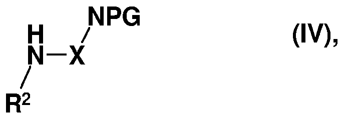 wherein R² and X are defined as in one of claims 1 to 6 and NPG denotes a protected or masked amino functionality, the derivatives or salts thereof, or
b) a compound of general formula 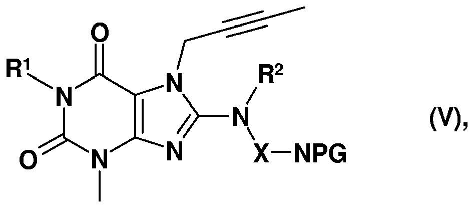 wherein R¹, R² and X are defined as in one of claims 1 to 6 and NPG denotes a protected or masked amino functionality, is deprotected,
and/or
any protecting groups used during the reaction are then cleaved and/or
the compounds of general formula I thus obtained are resolved into their enantiomers and/or diastereomers and/or
the compounds of formula I thus obtained are converted into their salts, particularly for pharmaceutical use into the physiologically acceptable salts thereof with inorganic or organic acids or bases.

## Revendications

1. Composés de formule générale 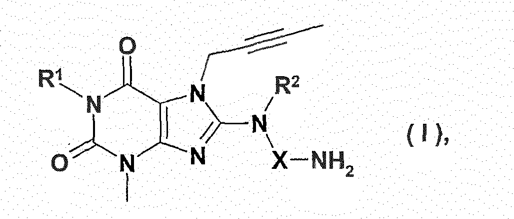 dans laquelle
R¹ représente un groupe hétéroarylméthyle ou hétéroaryléthyle,
un groupe hétéroarylcarbonylméthyle ou
un groupe arylprop-2-ènyle ou hétéroarylprop-2-ènyle, dans lesquels la chaîne propènyle peut être substituée par 1 à 4 atomes de fluor ou un groupe cyano, (alcoxy en C₁₋₃)carbonyle ou nitro,
R² représente un groupe alkyle en C₁₋₄ qui peut être linéaire ou ramifié, et
X représente un groupe -CH₂CH₂- qui peut être éventuellement substitué par un ou deux groupes alkyles en C₁₋₃, pouvant être identiques ou différents,
où, sauf stipulation contraire, les groupes alkyles, alcényles et alcinyles susmentionnés peuvent être linéaires ou ramifiés,
leurs tautomères, énantiomères, diastéréomères, leurs mélanges, leurs prodrogues et leurs sels.

2. Composés de formule générale I selon la revendication 1, dans lesquels
R¹ est tel que défini dans la revendication 1,
R² représente un groupe méthyle ou éthyle et
X représente un groupe -CH₂CH₂-, qui peut être éventuellement substitué par un ou deux groupes méthyles ou éthyles, les substituants pouvant être identiques ou différents,
leurs tautomères, énantiomères, diastéréomères, leurs mélanges, et leurs sels.

3. Composés de formule générale I selon la revendication 2, dans lesquels
R¹ représente un groupe phénylprop-2-ènyle, pyridinylméthyle, pyrimidinylméthyle, quinolinylméthyle, quinolinonylméthyle, imidazoquinolinylméthyle, isoquinolinylméthyle, quinazolinylméthyle, quinazolinonylméthyle, quinoxalinylméthyle, phénanthridinylméthyle, naphthyridinyl méthyle, benzonaphthyr_idinylméthyle, imidazopyridinylméthyle ou benzotriazolylméthyle, qui peuvent être substitués respectivement par un ou deux atomes de fluor, de chlore, de brome ou un ou deux groupes cyano, nitro, amino, alkyle en C₁₋₃, alkyloxy en C₁₋₃, phényle et morpholinyle, les substituants pouvant être identiques ou différents,
R² représente un groupe méthyle et
X représente un groupe -CH₂CH₂- qui peut être éventuellement substitué par un ou deux groupes méthyles,
leurs tautomères, énantiomères, diastéréomères, leurs mélanges, et leurs sels.

4. Composés de formule générale I selon la revendication 3, dans lesquels
R¹ représente un groupe pyridinylméthyle, pyrimidinylméthyle, quinolinylméthyle, isoquinolinylméthyle, quinazolinylméthyle, quinoxalinylméthyle, naphthyridinylméthyle, ou naphthylméthyle, qui peuvent être substitués respectivement par un ou deux groupes cyano ou méthyle,
R² est un groupe méthyle et
X représente un groupe -CH(CH₃)-CH₂-, un groupe -CH₂-CH(CH₃)- ou un groupe -CH₂-C(CH₃)₂-,
leurs tautomères, énantiomères, diastéréomères, leurs mélanges, et leurs sels.

5. Composés de formule générale I selon la revendication 4, dans lesquels
R¹ représénte un groupe pyridinylméthyle, isoquinolinylméthyle, quinazolinylméthyle, quinoxalinylméthyle, ou naphthyridinylméthyle, qui peuvent être substitués respectivement par un groupe cyano ou méthyle,
R² représente un groupe méthyle et
X représente un groupe -CH(CH₃)-CH₂-, un groupe -CH₂-CH(CH₃)- ou un groupe -CH₂-C(CH₃)₂-, où respectivement l'atome de carbone situé à droite est relié au groupe amino de fin de terminaison,
leurs tautomères, énantiomères, diastéréomères, leurs mélanges, et leurs sels.

6. Composés suivants selon la revendication 1 :
(a) 1-[(3-méthylisoquinolin-1-yl)méthyl]-3-méthyl-7-(2-butin-1-yl)-8-[(2-aminoéthyl)-méthylamino]-xanthine
(b) 1-[(4-méthylquinazolin-2-yl)méthyl]-3-méthyl-7-(2-butin-1-yl)-8-[(2-aminoéthyl)-méthylamino]-xanthine
(c) 1-[(3-méthylisoquinolin-1-yl)méthyl]-3-méthyl-7-(2-butin-1-yl)-8-[(2-amino-2-méthyl-propyl)-méthylamino]-xanthine
(d) 1-[(4-méthylquinazolin-2-yl)méthyl]-3-méthyl-7-(2-butin-1-yl)-8-[(2-amino-2-méthyl-propyl)-méthylamino]-xanthine
(e) 1-[(3-méthylisoquinolin-1-yl)méthyl]-3-méthyl-7-(2-butin-1-yl)-8-[(S)-(2-aminopropyl) méthylamino]-xanthine
(f) 1-[(3-méthylisoquinolin-1-yl)méthyl]-3-méthyl-7-(2-butin-1-yl)-8-[(R)-(2-aminopropyl)-méthylamino]-xanthine
(g) 1-[(4-méthylquinazolin-2-yl)méthyl]-3-méthyl-7-(2-butin-1-yl)-8-[(S)-(2-aminopropyl)-méthylamino]-xanthine
(h) 1-[(4-méthylquinazolin-2-yl)méthyl]-3-méthyl-7-(2-butin-1-yl)-8-[(R)-(2-aminopropyl)-méthylamino]-xanthine
(i) 1-[(3-méthylisoquinolin-1-yl)méthyl]-3-méthyl-7-(2-butin-1-yl)-8-[(R)-(2-amino-1-méthyléthyl)-méthylamino]-xanthine
(j) 1-[(3-méthylisoquinolin-1-yl)méthyl]-3-méthyl-7-(2-butin-1-yl)-8-[(S)-(2-amino-1-méthyléthyl)-méthylamino]-xanthine
(k) 1-(2-cyanobenzyl)-3-méthyl-7-(2-butin-1-yl)-8-[(S)-(2-aminopropyl)-méthylamino]-xanthine
(l) 1-[([1,5]naphthyridin-2-yl)méthyl]-3-méthyl-7-(2-butin-1-yl)-8-[(S)-(2-aminopropyl)-méthylamino]-xanthine
(m) 1-[(quinoxalin-6-yl)méthyl]-3-méthyl-7-(2-butin-1-yl)-8-[(S)-(2-aminopropyl)-méthylamino]-xanthine
(n) 1-[(3-cyanopyridin-2-yl)méthyl]-3-méthyl-7-(2-butin-1-yl)-8-[(S)-(2-aminopropyl)-méthylamino]-xanthine
(o) 1-[(4-cyano-isoquinolin-3-yl)méthyl]-3-méthyl-7-(2-butin-1-yl)-8-[(S)-(2-aminopropyl)-méthylamino]-xanthine
(q) 1-[(3-cyanopyridin-2-yl)méthyl]-3-méthyl-7-(2-butin-1-yl)-8-[(2-amino-2-méthylpropyl)-méthylamino]-xanthine
et leurs sels.

7. Sels physiologiquement acceptables des composés selon l'une des revendications 1 à 6 avec des acides ou des bases, minéraux ou organiques.

8. Médicament contenant un composé selon l'une des revendications 1 à 6 ou un sel physiologiquement acceptable selon la revendication 7, en plus éventuellement d'un ou de plusieurs véhicules et/ou diluants inertes.

9. Utilisation d'un composé selon au moins l'une des revendications 1 à 7 pour la préparation d'un médicament, qui est approprié au traitement du diabète sucré de type 1 et II, de l'arthrite, de l'adiposité, de la transplantation d'allo-greffe et de l'ostéoporose provoquée par la calcitonine.

10. Procédé de préparation d'un médicament selon la revendication 8, **caractérisé en ce qu'**un composé selon au moins l'une des revendications 1 à 7 est incorporé par voie non chimique dans un ou plusieurs véhicules et/ou diluants inertes :

11. Procédé de préparation des composés de formule générale I selon les revendications 1 à 7, **caractérisé en ce que**
a) l'on fait réagir un composé de formule générale I 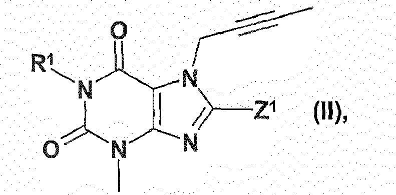 dans laquelle
R¹ est tel que défini dans l'une des revendications 1 à 6, et
Z¹ représente un groupe sortant comme un atome d'halogène, un groupe hydroxy, mercapto, sulfinyle, sulfonyle ou sulfonyloxy substitué,
avec un composé de formule générale 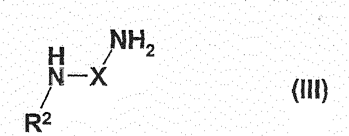 ou 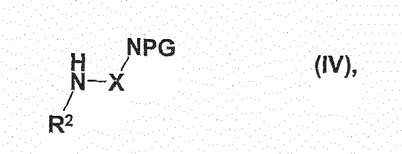 dans lesquelles R² et X sont tels que définis dans l'une des revendications 1 à 6 et NPG représente une fonctionnalité amino protégée ou masquée, ses dérivés ou sels, ou
b) on déprotège un composé de formule générale 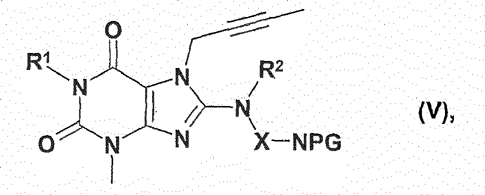 dans laquelle R¹, R² et X sont tels que définis dans l'une des revendications 1 à 6 et NPG représente une fonctionnalité amino protégée ou masquée,
et/ou
l'on sépare ensuite éventuellement les groupes protecteurs utilisés pendant la réaction et/ou l'on sépare les composés ainsi obtenus de formule générale I en leurs énantiomères et/ou diastéréomères et/ou
l'on transforme les composés obtenus de formule I en leurs sels, en particulier pour l'utilisation pharmaceutique en leurs sels physiologiquement acceptables, avec des acides ou des bases, minéraux ou organiques.
